# EUROPEAN PATENT APPLICATION

(11) **EP 3 808 372 A1**
(43) Date of publication of application: **21.04.2021**
(21) Application number: 19203834.7
(22) Date of filing: 17.10.2019
(51) Int. Cl.: A61K 39/00, A61P 31/04, A61K 39/112

(54) **NOVEL VACCINE COMPOSITIONS**

(71) Applicant: GlaxoSmithKline Biologicals S.A., 1330 Rixensart (BE)
(72) Inventor: The designation of the inventor has not yet been filed
(74) Representative: Sanderson, Andrew John

(57) **Abstract**

A *Shigella flexneri* O-antigen of a first serotype or subserotype for use in raising an immune response against one or more *Shigella flexneri* O-antigen of a different serotype or subserotype, together with associated binding moieties, pharmaceutical compositions, kits, uses or methods.

## Description

### BACKGROUND

*Shigella* infections are endemic throughout the world, but the main disease burden is in developing countries. The Global Burden of Disease Study 2017 estimates that *Shigella* causes 15.2% (i.e. 238,000) of the 1.57 million deaths caused by diarrheal infections (1) with 98.5% of *Shigella* deaths occurring in low and middle income countries. Children younger than 5 years of age accounted for 33% of deaths. Consistent with these global estimates, the prospective Global Enteric Multicenter Study (GEMS) found that shigellosis is one of the top causes of moderate to severe diarrhoea (MSD) in children under 5-year-old in 7 sites in sub-Saharan Africa and South Asia (2). Of 1120 isolates typed, *S*. *sonnei* was the dominant species but a range of *S*. *flexneri* serotypes was found in the different sites. Overall, the dominant *S*. *flexneri* serotype was *S*. *flexneri* 2 but the distribution of the serotypes and subtypes varied according to location. For example, in the Bangladesh sites the order was *S*. *flexneri* 2a, 2b, 3a, 6, 1b, 4a and Y (X was not detected); in Kenya the order instead was 6, 1b, 3a, 4a, 2b and 2a (1a, X and Y, were not detected).

A comprehensive literature survey of 16,587 reported cases in low and middle-income counties extended the GEMs results to 35 countries (3). These data, even aggregated at the WHO regional level, show major differences in the serotype frequencies: *S*. *flexneri* 2 was the most common serotype in the African (AFRO), American (AMRO), South-East Asian (SEARO) and Western Pacific (WPRO) Regions; but *S*. *flexneri* 6 was the most common in the Eastern Mediterranean Region (EMRO). The second most common serotype was more variable: *S*. *flexneri* 1 in AFRO, *S*. *flexneri* 2 in EMRO, *S*. *flexneri* 3 in SEARO, both *S*. *flexneri* 3 and *S*. *flexneri* 4 in AMRO and *S*. *flexneri* 4 in WPRO. Even without considering the less frequent serotypes (that still cause a significant part of the disease burden), these data highlight the difficulties with making a broadly specific vaccine if based on the serotype or subtype specific immunogens.

*Shigella* vaccines under development span a spectrum of approaches and antigens (4, 5). Almost all *Shigella* vaccines include the O Antigen (OAg) component of the lipopolysaccharide (LPS), which is considered a protective antigen (6) but this antigen would restrict vaccine efficacy to (i) homologous protection or (ii) those cross-reactions with other serotypes capable of conferring protection, that were defined by the OAg alone.

All serotypes and sub-serotypes of *S*. *flexneri,* except serotype 6, share a common OAg backbone (7, 8) with repeats of:

→ 2)-α-L-Rha*p*^{III}-(1→2)-α-L-Rha*p*^{II}-(1→3)-α-L-Rha*p*^{I}-(1→3)-β-D-Glc*p*NAc-(1→

The addition of glucosyl or O-acetyl residues to the backbone sugars creates the OAg structures specific to the different serotypes. The enzymes responsible for the modification of the backbone are encoded on mobile elements and new *S*. *flexneri* serotypes and subtypes could emerge by bacteriophage-mediated integration of OAg modification genes (9, 10). The serotypes *S*. *flexneri* 1, 2, 3, 4, 5 and X are defined by type specificities (I, II, III, IV, V and X) created by glucosylation (serotypes I, II, IV, V and X). Type specificity III (*S*. *flexneri* 3) is defined by acetylation on rhamnose I and an absence of glucosylation that defines other type specificities. *S*. *flexneri* Y does not contain any of these substitutions and is defined by the absence of the serotype specificities. The polysaccharide present in serotype Y is characterized by two antigenic specificities labelled dual group O-factor 3,4. A structural domain that defines this O-factor has not been completely identified yet. In some cases, its manifestation is ambiguous as strains otherwise identical in the O-antigen structure and the presence of other immunodeterminants may express or may not express O-factor 3,4 (e.g. former serotypes 3b and 3c, which have been proposed to be combined into one serotype 3b [10]). The 3,4 factor is related to the structure, so can be masked by other specificities. The polysaccharide can be modified by adding various chemical groups (*α*-D-glucopyranosyl, O-acetyl, phosphoethanolamine) to different sugars giving rise to enormously diverse O-antigen structures and, correspondingly, to serological heterogeneity, which is the basis for serotyping of *S*. *flexneri* strains (11). *S*. *flexneri* 6 does not share the common backbone. Instead it has two repetitions (11) of rhamnose, one galacturonic acid and one N-acetylgalactosamine (8).

→ 2)-α-L-Rha*p*^{III}-(1→2)-α-L-Rha*p*^{II}-(1→4)-β-D-Gal*p*A-(1→3)-β-D-Gal*p*NAc-(1→

Although phylogenetically dissimilar (*S*. *flexneri* 6 is in the *S*. *boydii* cluster) (12), *S*. *flexneri* 6 reacts with *S*. *flexneri* species-specific antisera, possibly because of the similarity of the trisaccharide →3)-β-D-Gal*p*NAc-(1→2)-a-L-Rha*p*^{III}-(1→,2)-α-L-Rha*p*^{II}-(1→ that crosses the junction between adjacent repeats in other *S*. *flexneri* serotypes.

All serotypes of *S*. *flexneri* (including serotype *S*. *flexneri* 6) can have additional modifications, involving substitution with glucose, acetate or phosphoethanolamine, that are common to several of the serotypes and generate the group specificities 6; 7,8; 9; 10; IV-1 or have the group specificity 3,4 that is part of the unmodified repeating unit. The O-antigens of *S*. *flexneri* non-6 serotypes are highly diverse due to various chemical modifications to the basal structure giving rise to the observed serological heterogeneity. Several genes outside the O-antigen cluster are involved in the modifications, which occur after the O-unit assembly and before the transfer of the mature O-polysaccharide to the lipid A-core region of the LPS. The O-antigen plays an important role in the pathogenesis of *S*. *flexneri;* particularly, it protects the bacteria from the lytic action of serum complement and promotes adherence and internalization of bacteria to intestinal epithelial cells. Creating antigenic diversity by O-antigen modifications is considered as an important virulence factor of *S*. *flexneri* that enhances survival of the pathogens because the host must mount a specific immune response to each serotype. Moreover, such modification as glucosylation at certain sites promotes invasion of *S*. *flexneri* into host cells mediated by the type III secretion system [11].

These type- and group-specificities are present in various combinations (at least 31 have been reported to date) (11) and generate shared epitopes that are potential targets of antibodies elicited by cross-reacting vaccines.

This was the basis of the studies by Noriega *et al.,* (13) who tested a bivalent vaccine of *S*. *flexneri* 2a and *S*. *flexneri* 3a in challenge studies in guinea pigs, which was predicted to protect against most isolates via their group specificities. In a conjunctivitis model, this achieved protection against serotypes 1b, 2b, 5b and Y (as predicted based on shared group specificities) but not for serotypes 1a and 4b (contrary to the prediction). Noriega et *al.,* predicted that this vaccine would have not protected against *S*. *flexneri* 6. However, subsequent work has identified epitope 9 which is common to all known *S*. *flexneri* 6 and some *S*. *flexneri* 2a isolates (11). Thus, if protection can be mediated through group specificities, and if the *S*. *flexneri* 2a isolate used in Noriega *et al.,* had a 9 specificity, it could have been expected to be protective.

Thus, the identification of a shared group specificity between *S*. *flexneri* 6 and *S*. *flexneri* 2a suggests that group specificities are not predictive of cross-protection since, if they were, Noriega et *al.,* would have seen cross-protection of *S*. *flexneri* 6 through vaccination with *S*. *flexneri* 2a O-antigen. Contrary to the prediction of Noriega *et al.,* the present inventors have surprisingly found that *S*. *flexneri* 2a does not protect against serotype 1b (Table 2 herein). Further, while the hypothesis of Noriega *et al.,* is that *S*. *flexneri* 3a would confer cross-protection against serotypes 1b, 2b, 5b and Y, Table 2 herein surprisingly shows that protection is not conferred against *S*. *flexneri* 2b. Noriega *et al.,* were unable to detect these absences of predicted cross-protection because they did not perform control vaccinations with serotype 2a alone, and serotype 3a alone. Only vaccination with serotypes 2a and 3a in combination was performed, masking gaps in the predicted cross-protection.

To our knowledge, outside of Noriega *et al.,* evidence for cross-protection is limited in the literature to:
Farzam *et al.,* 2017 (31) found that mice immunised with a *S*. *flexneri* 2a O-antigen conjugate did not elicit antibody that reacted with *S*. *flexneri* 6 O-antigen, while sera from humans immunised with the *S. flexneri* 2a O-antigen conjugate did elicit antibody that bound to *S*. *flexneri* 6 O-antigen but concluded that *"[t]he S. flexneri 2a vaccine will not suffice by itself to protect against both types"* (see page 4995, right column, second full paragraph, lines 4-5). Interestingly, the present inventors have found that vaccination with *S*. *flexneri* 2a did not induce killing of *S*. *flexneri* 6 in serum bactericidal assay (SBA), suggesting the protection against *S*. *flexneri* 6 infection was mediated by a means other than complement mediated serum killing.
Ferrecio et *al.,* 1991 (37) followed a cohort of young children during an initial phase covering 8609 child months of observation in which 87 children has a first episode of *S*. *sonnei, S. flexneri* 2a or *S*. *flexneri* 6 infection. New infections were recorded in a follow up of 1200 child months of these 87. The authors concluded that there is strain-specific protection against *S*. *sonnei, S. flexneri* 2a and *S*. *flexneri* 6 but no cross-protection of other serotypes following natural infection. An insufficient number of infections with any *S*. *flexneri* serotype arose in the study to give a statistically valid indication of level of protection. The S. flexneri 6 infection levels came closest to statistical significance but suggested that *S*. *flexneri* 2a does not protect against *S*. *flexneri* 6. There were so few *S*. *flexneri* infections of other serotypes that it was impossible to conclude if *S*. *flexneri* 2a could have had any impact on other *S*. *flexneri* serotypes.
Mel *et al.,* 1971 (38) was a field trial of two bivalent live oral vaccines. Vaccine A combined *S*. *flexneri* 1 with *S*. *flexneri* 2a. Vaccine B combined *S*. *flexneri* 3 with *S*. *sonnei.* There were no controls. The implicit assumption in the vaccine trial design is that the immunity is serotype-specific since efficacy was judged by the difference in cases of each detected serotypes in the two vaccines. The data suggests that vaccine A was effective against *S*. *flexneri* 1 with *S*. *flexneri* 2a (as expected), but vaccine B could only be demonstrated against *S*. *sonnei.* Since there was only one case of *S. flexneri* 3 detected in the vaccine A group, but three in the vaccine B group the results for S. flexneri 3 are inconclusive.
Karnell *et al.,* 1992 (39) vaccinated Rhesus monkeys by infection with an attenuated Y strain and reported them to be protected against subsequent challenge with virulent *S*. *flexneri* 1b, 2a and Y (n.b., *S*. *flexneri* Y comprises the base O-antigen structure of all *S*. *flexneri* except serotype 6). However, all monkeys had significant pre-vaccination titres to *S*. *flexneri* 1, 2a and Y LPS, raising the prospect that the protection was due to non-specific factors arising from pre-study infection(s). Since they saw no serotype specificity in the protection, any protection elicited by their vaccine appeared to be mediated by something other than O-antigen. One possibility is outer-membrane protein conserved between serotypes.

While it was encouraging to see some cross-protection in Noriega *et al.,* even so, its results suggest that the envisaged coverage of this binary combination in the field is limited since globally, *S*. *flexneri* 1b, 4b and 6 are responsible for approximately equal burden of disease, ranking behind *S*. *flexneri* 2a and 3a. To address this, Livio et *al.,* proposed including *S*. *flexneri* 6 in a combination vaccine (14). However, this would still leave people unprotected against *S*. *flexneri* 4, the second most common *S*. *flexneri* serotypes in the Americas and the Western Pacific region (3). Based on the Noriega et *al.,* data, *S*. *flexneri* 4b was not protected by the *S*. *flexneri* 2a/3a combination in guinea pigs, and these authors assume that *S*. *flexneri* 6 does not cross protect against *S*. *flexneri* 4b.

Since shared group-specificity was found not to be predicative of cross-protection it was unclear which cross-reactions would result in cross-protection and, consequently, how many strains must be represented in a vaccine to obtain sufficient strain coverage to be viable. This is a critical question because vaccine cost increases with complexity and, since *S*. *flexneri* is predominantly a disease of developing countries, its treatment requires a low a cost of goods to be economically viable. The lower the cost of goods, the greater the impact a vaccine can have on global health. Hence, it is important to establish which cross-reactions would result in cross-protection.

However, to our knowledge the only publications providing experimental evidence on the presence or absence of *S.flexneri* cross protection (as opposed to cross-reaction) largely indicate that shared group-specificity does not confer cross-protection.

Hence, there are cross-reactions identified by FACS and cross-protections identified by SBA that cannot be predicted by combination of known group- or type-specificities. This may be explained by the way serogroups and serotypes are defined. The *Shigella* serogroups and serotypes are identified by agglutination tests with polyclonal antibodies, where a positive interaction indicates that a strain contains antigens that specifically react with the serum antibodies.

These polyclonal antisera are obtained by hyperimmunization of healthy rabbits with heat-inactivated whole cells of *S*. *flexneri* (40). The antisera are absorbed against cells of other *S*. *flexneri* serotypes (and/or subserotypes) to remove cross-reacting agglutinins and thereby create serotype- or subserotype-specific antisera.

There are polyvalent and monovalent *Shigella* antisera. The polyvalent antisera are those that their antibodies recognise antigens present in the different serotypes of *Shigella* (e.g., polyvalent antisera for *S.flexneri,* recognises all the serotypes of this group). The monovalent antisera only recognise specific epitopes of a serotype (e.g., monovalent antisera for *S*. *flexneri* 2) or of a group factor (e.g., monovalent antisera for *S*. *flexneri* group factor 7,8). There are probably a multitude of epitopes not covered by the typing scheme currently in use.

The specificity of the antisera used in typing reactions disguises that non-absorbed antisera are not serotype- or group-factor-specific and contain agglutinins for other serotypes which are removed for serotyping. Hence, although cross-reacting agglutinins are removed (or reduced to a concentration that cannot induce agglutination in the slide agglutination test), cross-reacting antibodies that do not induce agglutination are retained. Moreover, sera produced in vaccine trials are not absorbed against other strains and so retain all cross-reacting agglutinins.

However, as is clear from references 11, 13, 31 and 37-39 (*supra.*) these cross-reactive antibodies do not necessarily confer cross-protection and, where observed, cross-protection does not appear to correlate with serotype or sub-serotype.

Three Gtr proteins (GtrA, GtrB, and type-specific Gtr (Gtr(type)) mediate glucosylation of the O-polysaccharide backbone. A single operon on the chromosome encoding Gtr proteins (gtr cluster) is carried by a (cryptic) prophage acquired by lysogeny of the bacteria with one or two from five temperate bacteriophages (Sfl, Sfll, SfIV, SfV, and SfX). All bacteriophages have been isolated from the corresponding *S.flexneri* strains and well characterised. Lysogeny with bacteriophages Sfl, Sfll, SfIV, SfV, and SfX converts serotype Y to serotypes 1a, 2a, 4a, 5a, and X, respectively, whereas the potential recipient range among other serotypes is quite different. The limitation in the host recognition is evidently due to the phage immunity from a modified O-antigen, which constitutes the receptor for the phage adsorption on the cell surface, a mechanism by which lysogeny prevents subsequent infection of bacteria by homologous or related phages, providing an evolutionary advantage to phages. Similarly, genes for O-Acetylation of Rhal by an acetyltransferase and phosphorylation with PEtN groups to Rhall or/and Rhalll are/were also provided by bacteriophage.

Antigenic diversity by O-antigen modifications is considered as an important virulence factor of *S*. *flexneri* that enhances survival of the pathogens because the host must mount a specific immune response to each serotype. Moreover, such modification as glucosylation at certain sites promotes invasion of *S*. *flexneri* into host cells mediated by the type III secretion system.

Accordingly, there remains a continuing need to determine which, if any, *S*. *flexneri* serotype and/or subserotype cross-reactions result in cross protection to determine the number and identity of serotypes and/or subserotypes that must be represented in a vaccine to provide acceptable coverage at an acceptable cost.

### DESCRIPTION

The present inventors examined the ability of sera raised against 14 subtypes of *S*. *flexneri* to (a) bind to a panel of 11 *S. flexneri* subtypes from all serotypes using fluorescence-activated cell sorting (FACS); and (b) to kill these bacteria in a complement-mediated serum bactericidal assay (SBA). The antigens were delivered as GMMA (Italian *gemma* = bud), which are outer membrane blebs of approximately 50-200 nm that bud off Gram-negative bacteria genetically modified to induce hyperblebbing (15), a technology currently in human vaccine trials for *S*. *sonnei* (16-18). GMMA contain outer-membrane components of the parent bacteria including the LPS expressing the OAg (19).

As mentioned, a broadly-protective vaccine against shigellosis needs to cover multiple *S*. *flexneri* serotypes. A challenge is to design a practical vaccine that balances coverage versus complexity and cost. Importantly, the present inventors found that a simple three-component vaccine of GMMA from *S*. *sonnei, S. flexneri* 1b and 3a would induce killing of most epidemiologically significant *Shigella* strains. This was not predicted based on cross-reactivity of currently-described shared serotypes and serogroups. The study presented here provides a framework for empirically designing such a vaccine.

There was strong cross-reaction within serotypes, e.g., sera raised against *S*. *flexneri* 2a reacted strongly with *S. flexneri* 2b. We identified some immunogens (e.g. *S*. *flexneri* 1b and 3a) that induced broadly-reactive antibodies that bound to most of the *S*. *flexneri* in the panel, while other immunogens (e.g., *S*. *flexneri* 2a) had a narrower specificity. Contrary to expectation, most cross-reactions cannot be assigned to *S*. *flexneri* serogroups e.g., sera raised with *S*. *flexneri* 1b strongly reacted with *S*. *flexneri* 6 which do not share any of the currently recognised serogroups. These results suggest that there are common groups specificities not currently recognised with typing reagents and that broadly cross-reactive vaccines will be possible with limited components (e.g., just *S*. *flexneri* 1b and 3a).

Accordingly, a first aspect of the invention provides a *Shigella flexneri* O-antigen of a first serotype or subserotype for use in raising an immune response against one or more *Shigella flexneri* O-antigen of a different serotype or subserotype.

Lipopolysaccharides (LPS), also known as lipoglycans and endotoxins, are large molecules consisting of a lipid and a polysaccharide composed of O-antigen, outer core and inner core joined by a covalent bond and are found in the outer membrane of Gram-negative bacteria. A repetitive glycan polymer contained within an LPS is referred to as the O antigen, O polysaccharide, or O side-chain of the bacteria. The O antigen is attached to the core oligosaccharide and comprises the outermost domain of the LPS molecule. The composition of the O chain varies from strain to strain. The Core domain always contains an oligosaccharide component that attaches directly to lipid A and commonly contains sugars such as heptose and 3-Deoxy-D-manno-oct-2-ulosonic acid (also known as KDO, keto-deoxyoctulosonate). Lipid A is, in normal circumstances, a phosphorylated glucosamine disaccharide decorated with multiple fatty acids. These hydrophobic fatty acid chains anchor the LPS into the bacterial membrane, and the rest of the LPS projects from the cell surface. The lipid A domain is responsible for much of the toxicity of Gram-negative bacteria.

By "a *Shigella flexneri* O-antigen of a first serotype" we mean or include complete O-antigen, or fragments, fusions and/or derivatives thereof. The O-antigen may or may not be bound to the LPS core domain. The LPS core domain may or may not be bound to lipid A. Hence, the O-antigen may comprise part of a complete LPS molecule. By "fragment" of an O-antigen, we mean or include molecules that comprise or consist of at least 25% of the contiguous length of a reference O-antigen molecule e.g., at least 50%, at least 75%, at least 90%, at least 95%, at least 98% or at least 99% of the contiguous length of a reference O-antigen molecule. When referring to "a *Shigella flexneri* O-antigen of a first serotype" in the singular, as is convention in patent drafting, we mean or include pluralities of the same O-antigen. Alternatively or additionally, we mean or include single O-antigen molecules. By "first serotype" we mean or include a single subserotype within the 'first serotype'; alternatively, we mean or include a mixture of serotypes within the 'first serotype', for example, 2, 3 or all of the serotypes within the 'first serotype'.

By "different serotype or subserotype" we mean or include another serotype or subserotype to the 'first serotype or subserotype'. For the avoidance of doubt, where there is more than one 'different serotype or subserotype', each 'different serotype or subserotype' is from a different serotype or subserotype to each other, as well as to the 'first serotype or subserotype'.

Alternatively or additionally, the immune response is raised against one or more *Shigella flexneri* O-antigen of a different serotype. By "a different serotype" we mean or include another serotype to the 'first serotype or subserotype'. Hence, the or each 'different serotype' is from a different serotype to the 'first serotype or subserotype'. This does not exclude that the O-antigen of a 'first serotype or subserotype' induces an immune response against the 'first serotype or subserotype' or against other subserotypes of the same serotype as the 'first serotype or subserotype', only that this subject-matter does not necessarily form part of the claimed subject-matter.

The SBAs of Table 2 indicate which other *S*. *flexneri* strains a first *S*. *flexneri* strain was capable of inducing complement-mediated killing against. To ensure that the claimed cross-protections were sufficiently strong to be biologically relevant for vaccinology, a minimum threshold SBA score of 2.3 was selected which represents a 200x increase from baseline. SBA scores of 3.0, 3.6 and 3.7 represent 400x, about 900x and 1000x increases from baseline, respectively and may be used as even more stringent SBA activity thresholds. Hence, alternatively or additionally, the different serotype or subserotype is one or more serotype or subserotype having an SBA score in Table 2 of greater than or equal to 2.3, for example, greater than or equal to 3.0, greater than or equal to 3.6, or greater than or equal to 3.7. Alternatively or additionally, the different serotype or subserotype is not one or more serotype or subserotype having an SBA score in Table 2 of less than 3.7, for example, less than 3.6, less than 3.0, or less than 2.3.

Hence, the present invention relates to the use of one or more O-antigen to induce an immune response against one or more further O-antigen and so, alternatively or additionally the first serotype or subserotype is:
1 and the one or more *S*. *flexneri* O-antigen of a different serotype or subserotype comprises or consists of a serotype or subserotype selected from the group consisting of serotype 1, 2, 3, 4, 5, 6, X and Y, for example, 1, 2, 3, 4, 5, 6, 7 or 8 of the different serotypes;
2 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
3 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
4 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
5 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
6 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
X and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes; and/or
Y and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes.

Alternatively or additionally, the first serotype or subserotype is:
1 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
2 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
3 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
4 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
5 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
6 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes;
X and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes; and/or
Y and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, X and Y, for example, 1, 2, 3, 4, 5, 6, 7, 8, 9, 10 or 11 of the different serotypes or subserotypes.

Alternatively or additionally, the first serotype or subserotype is:
1a and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
1b and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
2a and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
2b and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
3a and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
3b and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
4a and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
5b and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
6 and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
X and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes; and/or
Y and the one or more *S*. *flexneri* O-antigen of a different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes.

Alternatively or additionally, the first serotype or subserotype is:
1a and the one or more *S*. flexneri O-antigen of a different serotype or subserotype comprises or consists of a serotype or subserotype selected from the group consisting of serotype or subserotype 1b, 2a, 2b, 3a, 3b, 4a, 5b, 6, X and Y, for example, 1, 2, 3, 4, 5, 6, 7, 8, 9 or 10 of the different serotypes or subserotypes;
1b and the one or more *S*. *flexneri* O-antigen of a different serotype or subserotype comprises or consists of a serotype or subserotype selected from the group consisting of serotype or subserotype 1a, 2a, 2b, 3a, 3b, 4a, 5b, 6, X and Y, for example, 1, 2, 3, 4, 5, 6, 7, 8, 9 or 10 of the different serotypes or subserotypes;
2a and the one or more *S*. *flexneri* O-antigen of a different serotype or subserotype comprises or consists of a serotype or subserotype selected from the group consisting of serotype or subserotype 1a, 1b, 2b, 3a, 3b, 4a, 5b, 6, X and Y, for example, 1, 2, 3, 4, 5, 6, 7, 8, 9 or 10 of the different serotypes or subserotypes;
2b and the one or more *S*. *flexneri* O-antigen of a different serotype or subserotype comprises or consists of a serotype or subserotype selected from the group consisting of serotype or subserotype 1a, 1b, 2a, 3a, 3b, 4a, 5b, 6, X and Y, for example, 1, 2, 3, 4, 5, 6, 7, 8, 9 or 10 of the different serotypes or subserotypes;
3a and the one or more *S*. *flexneri* O-antigen of a different serotype or subserotype comprises or consists of a serotype or subserotype selected from the group consisting of serotype or subserotype 1a, 1b, 2a, 2b, 3b, 4a, 5b, 6, X and Y, for example, 1, 2, 3, 4, 5, 6, 7, 8, 9 or 10 of the different serotypes or subserotypes;
3b and the one or more *S*. *flexneri* O-antigen of a different serotype or subserotype comprises or consists of a serotype or subserotype selected from the group consisting of serotype or subserotype 1a, 1b, 2a, 2b, 3a, 4a, 5b, 6, X and Y, for example, 1, 2, 3, 4, 5, 6, 7, 8, 9 or 10 of the different serotypes or subserotypes;
4a and the one or more *S*. *flexneri* O-antigen of a different serotype or subserotype comprises or consists of a serotype or subserotype selected from the group consisting of serotype or subserotype 1a, 1b, 2a, 2b, 3a, 3b, 5b, 6, X and Y, for example, 1, 2, 3, 4, 5, 6, 7, 8, 9 or 10 of the different serotypes or subserotypes;
5b and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 6, X and Y, for example, 1, 2, 3, 4, 5, 6, 7, 8, 9 or 10 of the different serotypes or subserotypes;
6 and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, X and Y, for example, 1, 2, 3, 4, 5, 6, 7, 8, 9 or 10 of the different serotypes or subserotypes;
X and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6, and Y, for example, 1, 2, 3, 4, 5, 6, 7, 8, 9 or 10 of the different serotypes or subserotypes; and/or
Y and the one or more *S*. *flexneri* O-antigen of a different serotype or subserotype comprises or consists of a serotype or subserotype selected from the group consisting of serotype or subserotype 1a, 1b, 2a, 2b, 3a, 3b, 4a, 5b, 6 and X, for example, 1, 2, 3, 4, 5, 6, 7, 8, 9 or 10 of the different serotypes or subserotypes.

Alternatively or additionally, the first serotype or subserotype is from another serotype to the different serotype or subserotype, for example:
where the first serotype or subserotype is 1, the different serotype or subserotype is not, or is not a subserotype of, serotype 1;
where the first serotype or subserotype is 2, the different serotype or subserotype is not, or is not a subserotype of, serotype 2;
where the first serotype or subserotype is 3, the different serotype or subserotype is not, or is not a subserotype of, serotype 3;
where the first serotype or subserotype is 4, the different serotype or subserotype is not, or is not a subserotype of, serotype 4;
where the first serotype or subserotype is 5, the different serotype or subserotype is not, or is not a subserotype of, serotype 5;
where the first serotype or subserotype is 6, the different serotype or subserotype is not, or is not a subserotype of, serotype 6;
where the first serotype or subserotype is X, the different serotype or subserotype is not, or is not a subserotype of, serotype X;
where the first serotype or subserotype is Y, the different serotype or subserotype is not, or is not a subserotype of, serotype Y;

As noted in the introduction, the bivalent test vaccine of Noriega *et al.,* (13), and infection of monkeys of Karnell *et al.,* 1992 (39) reported potential cross-protections between *S*. *flexneri* strains. To our knowledge, there was no teaching as to whether these potential cross-protections were O-antigen- or protein-mediated.

Alternatively or additionally, where the first serotype or subserotype is:
2, the different serotype or subserotype is not, or is not a subserotype of, serotype 1;
2, the different serotype or subserotype is not, or is not a subserotype of, serotype 2;
2, the different serotype or subserotype is not, or is not a subserotype of, serotype 5;
2, the different serotype or subserotype is not, or is not a subserotype of, serotype Y;
3, the different serotype or subserotype is not, or is not a subserotype of, serotype 1;
3, the different serotype or subserotype is not, or is not a subserotype of, serotype 2;
3, the different serotype or subserotype is not, or is not a subserotype of, serotype 5;
3, the different serotype or subserotype is not, or is not a subserotype of, serotype Y;
2, and is provided in combination with an additional O-antigen of serotype or subserotype 3, the different serotype or subserotype is not, or is not a subserotype of, serotype 1;
2, and is provided in combination with an additional O-antigen of serotype or subserotype 3, the different serotype or subserotype is not, or is not a subserotype of, serotype 2;
2, and is provided in combination with an additional O-antigen of serotype or subserotype 3, the different serotype or subserotype is not, or is not a subserotype of, serotype 5;
2, and is provided in combination with an additional O-antigen of serotype or subserotype 3, the different serotype or subserotype is not, or is not a subserotype of, serotype Y;
2, the different serotype or subserotype is not, or is not a subserotype of, serotype 6;
Y, the different serotype or subserotype is not, or is not a subserotype of, serotype 1; and or
Y, the different serotype or subserotype is not, or is not a subserotype of, serotype 2.

Alternatively or additionally, where the first serotype or subserotype is:
2a, the different serotype or subserotype is not subserotype 1b;
2a, the different serotype or subserotype is not subserotype 2b;
2a, the different serotype or subserotype is not subserotype 5b;
2a, the different serotype or subserotype is not subserotype Y;
3a, the different serotype or subserotype is not subserotype 1b;
3a, the different serotype or subserotype is not subserotype 2b;
3a, the different serotype or subserotype is not subserotype 5b;
3a, the different serotype or subserotype is not, or is not subserotype Y;
2a, and is provided in combination with an additional O-antigen of serotype or subserotype 3a, the different serotype or subserotype is not subserotype 1b;
2a, and is provided in combination with an additional O-antigen of serotype or subserotype 3a, the different serotype or subserotype is not subserotype 2b;
2a, and is provided in combination with an additional O-antigen of serotype or subserotype 3a, the different serotype or subserotype is not subserotype b5;
2a, and is provided in combination with an additional O-antigen of serotype or subserotype 3a, the different serotype or subserotype is not, or is not subserotype Y; and/or
2a, the different serotype or subserotype is not, or is not subserotype 6;
Y, the different serotype or subserotype is not subserotype 1b; and or
Y, the different serotype or subserotype is not subserotype 2a.

Alternatively or additionally, the one or more O-antigen of a different serotype or subserotype does not share group specificities with the O-antigen(s) of the first serotype or subserotype.

By "does not share group specificities" we mean or include that the first and different O-antigens do not share group specifies as identified by typing reagents or genomic probes. Alternatively or additionally, the first and different O-antigens not share group specificities (3,4), 6, (7,8), 9 and 10 (or the structural modifications that determine these group specificities). By "the structural modifications" we mean or include:
Group specificity 6: *O-*acetylation of Rha*p*^{I} at position 2;
Group Specificity 7,8: α-D-glucopyranosyl substitution on position 3 of Rha*p*^{III}
Group Specificity 9: *O-*acetylation of Rha*p*^{III} at position 3 or 4 (3/4-*O*-acetylation); and/or
Group Specificity 10: *O-*acetylation of of GlcpNAc;
and wherein
Rha*p*^{I} is the L-Rhap attached in a 1->3 linkage to β-D-Glc*p*NAc;
Rhap" is the L-Rhap attached in a 1->3 linkage to α-L- Rha*p*^{I}; and/or
Rha*p*^{III} is the L-Rhap attached in a 1->2 linkage to α-L- Rha*p*^{III}.

The structural modification responsible for group 3,4 is not well defined. However, antibody typing sera is available that defines the presence or absence of the 3,4 specificity (3,4), 6, (7,8), 9 and 10, or the structural modifications that determine these group specificities (see, for example, Knirel et al., 2015, Biochemistry Moscow 'O-Antigen Modifications Providing Antigenic Diversity of Shigella flexneri and Underlying Genetic Mechanisms' 80(7):901-914, which is incorporated by reference herein, with particular reference to the structures listed in the table spanning pages 903-905).

As noted in the introduction, the limited cross-protection data available in the literature indicates that *S*. *flexneri* cross-protection cannot be predicted from known type- or group-specificities. Nevertheless, the present invention contemplates the inclusion of only those cross-protections that could not be predicted from the SBA scores of Table 2 that were based on shared group- and/or type-specificities. They may be defined by serotype versus serotype. Thus, alternatively or additionally, the first serotype or subserotype is:
serotype 1 and the different serotype or subserotype is one or more serotype selected from the group consisting of 2, 5 and X, for example, 1, 2 or 3 of the different serotypes.
serotype 2 and the different serotype or subserotype is one or more serotype selected from the group consisting of 4, 5, 6 and Y, for example, 1, 2 or 3 of the different serotypes.
serotype 3 and the different serotype or subserotype is one or more serotype selected from the group consisting of 1, 2, 4, 5, 6, X and Y, for example, 1, 2, 3, 4, 5, 6 or 7 of the serotypes;
serotype 4 and the different serotype or subserotype is one or more serotype selected from the group consisting of 1, 2, 5, X and Y, for example, 1, 2, 3, 4 or 5 of the serotypes;
serotype 5 and the different serotype or subserotype is one or more serotype selected from the group consisting of 1, 2, 4, 6, X and Y, for example, 1, 2, 3, 4, 5 or 6 of the serotypes;
serotype 6 and the different serotype or subserotype is one or more serotype selected from the group consisting of 5 and X, for example, 1 or 2 of the serotypes;
serotype X and the different serotype or subserotype is one or more serotype selected from the group consisting of 1, 4, 6 and Y, for example, 1, 2, 3 or 4 of the serotypes; and/or
serotype Y and the different serotype or subserotype is 5.

They may also be defined by serotype versus subserotype. Hence, Alternatively or additionally, the first serotype or subserotype is:
serotype 1 and the different serotype or subserotype is one or more subserotype selected from the group consisting of 2b, 5b and X, for example, 1, 2 or 3 of the different serotypes;
serotype 2 and the different serotype or subserotype is one or more serotype selected from the group consisting of 1a, 4a, 5b, 6 and Y, for example, 1, 2, 3, 4 or 5 of the different serotypes;
serotype 3 and the different serotype or subserotype is one or more serotype selected from the group consisting of 1a, 2a, 4a 5b, 6, X and Y, for example, 1, 2, 3, 4, 5, 6 or 7 of the serotypes;
serotype 4 and the different serotype or subserotype is one or more serotype selected from the group consisting of 1a, 2b, 5b, X and Y, for example, 1, 2, 3, 4 or 5 of the serotypes;
serotype 5 and the different serotype or subserotype is one or more serotype selected from the group consisting of 1a, 2a, 4a, 6 and Y, for example, 1, 2, 3, 4 or 5 of the serotypes;
serotype 6 and the different serotype or subserotype is one or more serotype selected from the group consisting of 5b and X, for example, 1 or 2 of the serotypes;
serotype X and the different serotype or subserotype is one or more serotype selected from the group consisting of 1a, 4a, 6 and Y, for example, 1, 2, 3 or 4 of the serotypes; and/or
serotype Y and the different serotype or subserotype is 5b.

They may further be defined by subserotype versus serotype. So, alternatively or additionally, the first serotype or subserotype is:
subserotype 1a and the different serotype or subserotype is one or more serotype selected from the group consisting of 5 and X, for example, 1 or 2 of the serotypes;
subserotype 1b and the different serotype or subserotype is one or more serotype selected from the group consisting of 2, 5 and X, for example, 1, 2 or 3 of the serotypes;
subserotype 1c and the different serotype or subserotype is one or more serotype selected from the group consisting of 2, 5 or X for example, 1, 2 or 3 of the serotypes;
subserotype 2a and the different serotype or subserotype is 1, 5 and Y for example, 1, 2 or 3 of the serotypes;
subserotype 2b and the different serotype or subserotype is one or more serotype selected from the group consisting of 4, 6 and Y, for example, 1 or 2 of the serotypes;
subserotype 3b and the different serotype or subserotype is one or more serotype selected from the group consisting of 1, 2, 4, 5, 6, X and Y, for example, 1, 2, 3, 4, 5, 6 or 7 of the serotypes;
subserotype 4a and the different serotype or subserotype is one or more serotype selected from the group consisting of 5 and X, for example, 1 or 2 of the serotypes;
subserotype 4b and the different serotype or subserotype is one or more serotype selected from the group consisting of 1, 2, 5, X and Y, for example, 1, 2, 3, 4 or 5 of the serotypes.
subserotype 5a and the different serotype or subserotype is X;
the first serotype is subserotype 5b and the different serotype or subserotype is one or more serotype selected from the group consisting of 1, 2, 4, 6, and Y, for example, 1, 2, 3, 4 or 5 of the serotypes;
serotype 6 and the different serotype or subserotype is one or more serotype selected from the group consisting of 5 and X, for example, 1 or 2 of the serotypes;
serotype X and the different serotype or subserotype is one or more serotype selected from the group consisting of 1, 4, 6 and Y, for example, 1, 2, 3 or 4 of the serotypes; and/or
serotype Y and the different serotype or subserotype is 5.

However, they may be defined by subserotype versus subserotype. Thus, alternatively or additionally, the first serotype or subserotype is:
subserotype 1a and the different serotype or subserotype is one or more subserotype selected from the group consisting of 5b and X, for example, 1 or 2 of the subserotypes;
subserotype 1b and the different serotype or subserotype is one or more subserotype selected from the group consisting of 2b, 5b and X, for example, 1, 2 or 3 of the subserotypes;
subserotype 1c and the different serotype or subserotype is one or more subserotype selected from the group consisting of 2b, 5b and X, for example, 1, 2 or 3 of the serotypes;
subserotype 2a and the different serotype or subserotype is subserotype 1a, 5b and Y, for example, 1, 2 or 3 of the subserotypes;
subserotype 2b and the different serotype or subserotype is one or more subserotype selected from the group consisting of 4a, 6 and Y, for example, 1, 2 or 3 of the subserotypes;
subserotype 3b and the different serotype or subserotype is one or more subserotype selected from the group consisting of 1a, 2a, 4a, 5b, 6, X and Y, for example, 1, 2, 3, 4, 5, 6 or 7 of the subserotypes;
subserotype 4a and the different serotype or subserotype is one or more subserotype selected from the group consisting of 5b and X, for example, 1 or 2 of the subserotypes;
subserotype 4b and the different serotype or subserotype is one or more subserotype selected from the group consisting of 1a, 2b, 5b, X and Y for example, 1, 2, 3, 4 or 5 of the subserotypes.
subserotype 5a and the different serotype or subserotype is X;
subserotype 5b and the different serotype or subserotype is one or more subserotype selected from the group consisting of 1a, 2a, 4a, 6 and Y, for example, 1, 2, 3, 4 or 5 of the subserotypes;
serotype 6 and the different serotype or subserotype is one or more subserotype selected from the group consisting of 5b and X, for example, 1 or 2 of the subserotypes;
serotype X and the different serotype or subserotype is one or more serotype selected from the group consisting of 1a, 4a, 6 and Y, for example, 1, 2, 3 or 4 of the subserotypes; and/or
serotype Y and the different serotype or subserotype is subserotype 5b.

Alternatively or additionally, the first serotype or subserotype is serotype 1 and the one or more different serotype or subserotype comprises or consists of one or more serotype selected from the group consisting of 2, 5, 6, X, and Y, for example 1, 2, 3,4 or 5 of these serotypes. Alternatively or additionally, the first serotype or subserotype is serotype 1 and the one or more different serotype or subserotype comprises or consists of serotype or subserotype 6. Alternatively or additionally, the first serotype or subserotype comprises or consists of 1a, 1b or 1c. Alternatively or additionally, the first serotype or subserotype is 1b.

Alternatively or additionally, the first serotype or subserotype is serotype 3 and the further serotype or subserotype is serotype 6. Alternatively or additionally, the first serotype or subserotype is one or more subserotype selected from the group consisting of 3a, 3b and 3c. Alternatively or additionally, the first serotype or subserotype is 3a.

Alternatively or additionally, the first serotype or subserotype is serotype 6 and the different serotype or subserotype is serotype 5. Alternatively or additionally, the different serotype or subserotype is one or more subserotype selected from the group consisting of 5a.

In contrast, the present invention also contemplates the exclusion of those cross-protections that could be predicted from the SBA scores of Table 2 that were based on shared group- and/or type-specificities. Hence, alternatively or additionally, the first serotype or subserotype is:
subserotype 1b and the different serotype or subserotype is not one or more subserotype selected from the group consisting of 3a and 3b, for example, 1 or 2 of the subserotypes;
subserotype 1c and the different serotype or subserotype is not 3a;
subserotype 2a and the different serotype or subserotype is not one or more subserotype selected from the group consisting of 3a and 6, for example, 1 or 2 of the subserotypes;
subserotype 2b and the different serotype or subserotype is not 3a;
subserotype 3a and the different serotype or subserotype is not one or more subserotype selected from the group consisting of 1a, 2b and X, for example, 1, 2 or 3 of the subserotypes;
subserotype 4a and the different serotype or subserotype is not one or more subserotype selected from the group consisting of 1b and 3a, for example, 1 or 2 of the subserotypes;
subserotype 4b and the different serotype or subserotype is not 1b.
subserotype 5a and the different serotype or subserotype is not one or more subserotype selected from the group consisting of 1b and 3a, for example, 1 or 2 of the subserotypes;
the first serotype is subserotype 5b and the different serotype or subserotype is not X;
serotype 6 and the different serotype or subserotype is not X;
serotype X and the different serotype or subserotype is not one or more serotype selected from the group consisting of 2b and 3a, for example, 1 or 2 of the subserotypes; and/or
serotype Y and the different serotype or subserotype is not one or more serotype selected from the group consisting of 1b, 2a and 3a, for example, 1, 2 or 3 of the subserotypes.

As discussed, an object of the present invention is to provide a broadly-protective vaccine against shigellosis that balances coverage versus complexity and cost. Accordingly, alternatively or additionally, the O-antigen of a first serotype is provided in combination with one or more additional O-antigen of a further serotype or subserotype, for example 1, 2, 3, 4, 5, 6, 7, 8, 9, 10, 11, 12, 13, 14 or 15 further O-antigen serotypes or subserotypes. For the avoidance of doubt, the first and additional O-antigens are of different subserotypes to one-another.

Alternatively or additionally, the first and further serotypes comprise or consist of combinations selected from the group consisting of:
a. serotype 1 (for example, subserotype 1a, 1b, or 1c) and serotype 3 for example, subserotype 3a, 3b, or 3c);
b. serotype 2 (for example, subserotype 2a, 2b, or 2c) and serotype 3 (for example, subserotype 3a, 3b, or 3c);
c. serotype 3 (for example, subserotype 3a, 3b, or 3c) and serotype 4 (for example, subserotype 4a, or 4b); and
d. serotype 3 (for example, subserotype 3a, 3b, or 3c) and serotype 5 (for example, subserotype 5a, or 5b).

Alternatively or additionally, the first and further subserotypes comprise or consist of combinations selected from the group consisting of:
a. 1b and 3a;
b. 1b and 3b;
c. 1c and 3a;
d. 1c and 3b;
e. 2a and 3b;
f. 3a and 4b; and
g. 3b and 5b.

Since the present invention seeks to provide a broadly-protective vaccine that balances coverage versus complexity and cost, where a first O-antigen serotype or subserotype protects against a further serotype or subserotype, alternatively or additionally, one or more of the different serotype(s) or subserotype(s) is not provided, for example, one or more of 1a, 1b, 1c (or 7a), 1d, 2a, 2b, 3a, 3b, 4a, 4av, 4b, 5a, 5b, X, Xv, Y, Yv, 6 and 7b is not provided, for example, 1, 2, 3, 4, 5, 6, 7, 8, 9, 10, 11, 12, 13, 14, 15, 16, 17 or 18 of the different serotype(s) or subserotype(s) is not provided.

Alternatively or additionally, the *Shigella flexneri* O-antigen for use is capable of raising an immune response against one or more of the different serotype(s) or subserotype(s) that is not provided. Alternatively or additionally, serotype 1 (for example, 1a, 1b, or 1c) is provided and serotype 6 is not provided. Alternatively or additionally, serotype 3 (for example, 3a, 3b, or 3c) is provided and serotype 6 is not provided. Alternatively or additionally, serotype 6 is provided and serotype 5 (for example, 5a or 5b) is not provided.

As mentioned, an object of the invention is to provide broad protection against shigellosis. Hence, alternatively or additionally, O-antigen from one or more *Shigella* species other than *Shigella flexneri* is provided in combination with the O-antigen of a first serotype. Alternatively or additionally, the one or more other *Shigella* species is selected from the group consisting of:
a. *Shigella sonnei;*
b. *Shigella boydii* (for example, serotype 1, 2, 3, 4, 5, 6, 7, 8, 9, 10, 11, 12, 13, 14, 15, 16, 17, 18, 19 or 20); and
c. *Shigella dysenteriae* (for example, serotype 1, 2, 3, 4, 5, 6, 7, 8, 9, 10, 11, 12, 13, 14 or 15).

Hence, alternatively or additionally, the *Shigella flexneri* O-antigen for use may comprise (e.g., may be provided with, either separately or as a mixture) O-antigen of *S. sonnei, S. boydii,* and *S*. *dysenteriae.* Alternatively or additionally, the *Shigella flexneri* O-antigen for use may comprise O-antigen of *S*. *sonnei* and *S. boydii.* Alternatively or additionally, the *Shigella flexneri* O-antigen for use may comprise O-antigen of *S. sonnei* and *S*. *dysenteriae.* Alternatively or additionally, the *Shigella flexneri* O-antigen for use may comprise O-antigen of *S*. *boydii,* and *S*. *dysenteriae.* Alternatively or additionally, the *Shigella flexneri* O-antigen for use may comprise O-antigen of *S*. *sonnei.* Alternatively or additionally, the *Shigella flexneri* O-antigen for use may comprise O-antigen of *S*. *boydii.* Alternatively or additionally, the *Shigella flexneri* O-antigen for use may comprise O-antigen of *S*. *dysenteriae.*

Alternatively or additionally, the *S*. *sonnei* is selected from the group consisting of *S*. *sonnei* , *S. sonnei* str. Moseley, *S*. *sonnei* 08-7761, *S*. *sonnei* 08-7765, *S*. *sonnei* 09-1032, *S*. *sonnei* 09-2245, *S*. *sonnei* 09-4962, *S*. *sonnei* 1DT-1, *S*. *sonnei* 3226-85, *S*. *sonnei* 3233-85, *S*. *sonnei* 4822-66, *S*. *sonnei* S6513 and *S*. *sonnei* Ss046.

Alternatively or additionally, two or more *Shigella flexneri* O-antigen types are provided in combination and comprise or consist of the group consisting of *Shigella flexneri* 1b, *Shigella flexneri* 2a, *Shigella flexneri* 3a, and *Shigella sonnei.*

Alternatively or additionally, the O-antigen is obtained or obtainable from a bacterial strain comprising an alteration that reduces lipopolysaccharide (LPS) toxicity (in particular, its pyrogenic potential). Alternatively or additionally, the lipopolysaccharide (LPS) expression modifying alteration reduces the toxicity of the *Shigella flexneri,* OMV released by it, and/or LPS produced by it, relative to the unaltered strain. Suitable methods for reducing toxicity and measuring that reduction are known, in the art, and can be found in, for example Rossi et al., 2014. Modulation of Endotoxicity of Shigella Generalized Modules for Membrane Antigens (GMMA) by Genetic Lipid A Modifications: Relative Activation of TLR4 and TLR2 Pathways in Different Mutants. J Biol. Chem., 289:24922-24935, which is incorporated by reference herein. Alternatively or additionally, the lipopolysaccharide (LPS) expression modifying alteration is induced by down-regulation, mutation or deletion (partial or complete) of one or more gene selected from the group consisting of:
- *msbB1* (IpxM) (lipid A biosynthesis myristoyltransferase);
- *msbB2* (IpxM) (lipid A biosynthesis myristoyltransferase);
- *htrB* (IpxL) (lipid A biosynthesis lauroyltransferase);
- *IpxP* (lipid A palmytoleoyl trasferase)
- *pagP* (adds palmitate to the primary linked acyl chain at 2-position Outer membrane);
- *IpxE* (removes 1-phosphate group);
- *IpxF* (removes 4 -phosphate group);
- *IpxO* (adds hydroxyl group to fatty acid myristate at 3 position);
- *IpxR* (removes acyl chain(s) from 3 position);
- *pagL* (removes acyl chain(s) from 3-position);

Alternatively or additionally, the O-antigen or LPS is obtained or obtainable from a bacterial strain modified to augment outer membrane vesicle (OMV) release. Strains of *Shigella flexneri, Shigella dysenteriae, Shigella boydii* and *Shigella sonnei* can be genetically modified to exhibit a hyper-blebbing phenotype by down-regulating or abolishing expression of one or more *tolR* or *OmpA.* Suitable mutations for down-regulating or abolishing expression include point mutations, gene deletions, gene insertions, and any modification of genomic sequences that results in a change in gene expression, particularly a reduction and more particularly inactivation or silencing.

The bacterium may be further genetically engineered by one or more processes selected from the following group: (a) a process of down-regulating expression of immunodominant variable or non-protective antigens, (b) a process of up-regulating expression of protective antigens, (c) a process of down-regulating a gene involved in rendering the lipid A portion of LPS toxic, (d) a process of up-regulating a gene involved in rendering the lipid A portion of LPS less toxic, and (e) a process of genetically modifying the bacterium to express a heterologous antigen.

Alternatively or additionally, one or more of the O-antigen(s) is/are provided:
a. unassociated with another macromolecule;
b. as a component of lipopolysaccharide (LPS), or a fragment thereof; or
c. conjugated to another macromolecule, for example, a protein (e.g., a carrier protein such as CRM197, tetanus toxoid, meningococcal outer membrane protein complex (OMPC), diphtheria toxoid, and *H. influenzae* protein D [see, for example, Pichichero, 2013, 'Protein carriers of conjugate vaccines Characteristics, development, and clinical trials' Hum. Vaccin. Immunother., 9(12):2505-2523, which is incorporated by reference herein]).

Alternatively or additionally, the protein is a carrier protein (i.e., proteins capable of increasing the potency of the immune response against polysaccharide or other polymer a conjugated to it).

Alternatively or additionally, the first serotype or subserotype, further serotype or subserotype and/or other *Shigella* species is/are provided as one or more membrane component, for example, a cell membrane (for example a Gram-negative bacterium cell membrane) or a vesicle membrane (for example, Gram-negative bacterium outer membrane vesicle [OMV]).

Alternatively or additionally, wherein the membrane component is obtained from a bacterial cell wherein at least 25% of the O-antigen is the same serotype as the O-antigen for use; for example, at least 35%, 50%, 60%, 70%, 80%, 90%, 95%, 96%, 97%, 98%, 99% or 100% of the O-antigen is the same serotype as the O-antigen for use.

The percentage of different O-antigen types present can be determined using any suitable means known in the art, such as the method taught in Micoli et al., 2018, 'Comparative immunogenicity and efficacy of equivalent outer membrane vesicle and glycoconjugate vaccines against nontyphoidal Salmonella' PNAS, 115(41): 10428-10433 which is incorporated by reference herein.

Alternatively or additionally, the bacterial cell is a strain selected from the group consisting of: *S. sonnei* 53G, *S. flexneri* 1b Stansfield, *S. flexneri* 2a 2457T, *S. flexneri* 2b 69/50, S. *flexneri* 3a str. 6885 and *S. flexneri* 6 str. 10.8537.

Alternatively or additionally, the membrane component is a component of an OMV selected from the group consisting of a detergent-extracted OMV (dOMV); or native OMV (nOMV).

Alternatively or additionally, the OMV is produced from genetically-modified bacterial strains that are mutated to enhance vesicle production and to remove or modify antigens (for example, lipid A).

*Shigella* bacteria used in the invention are, relative to their corresponding wild-type strains, hyperblebbing i.e. they release into their culture medium larger quantities of GMMA than the wild-type strain. These GMMA are useful as components of *Shigella* vaccines of the invention. The term GMMA is used to provide a clear distinction from conventional detergent-extracted outer membrane vesicles (dOMV), and native outer membrane vesicles (NOMV), which are released spontaneously from Gram-negative bacteria. GMMA differ in two crucial aspects from NOMV. First, to induce GMMA formation, the membrane structure has been modified by the deletion of genes encoding key structural components, specifically *tolR.* Second, as a consequence of the genetic modification, large quantities of outer membrane "bud off" (the Italian word for bud is 'gemma') to provide a practical source of membrane material for vaccine production, leading to increased ease of manufacturing and potential cost reduction. While NOMV have been used for immunogenicity studies, the yields are too low for practical vaccines.

*S. sonnei* GMMA used in the invention typically have a diameter of from 25 nm to 140 nm by electron microscopy, for example from 25nm to 40nm. GMMA may also have a bimodal size distribution. For example, the majority of GMMA having an average size from 25 nm to 40 nm in diameter (by EM) and a fraction of the particles having an average size from 65 nm to 140 nm. Particularly, at least 70%, at least 71%, at least 72%, at least 73%, at least 74%, at least 75%, at least 80%, at least 85%, at least 90% of the GMMA will have a diameter of from 25 nm to 140 nm.

GMMA are released spontaneously during bacterial growth and can be purified from the culture medium. The purification ideally involves separating the GMMA from living and/or intact *Shigella* bacteria, for example, by size-based filtration using a filter, such as a 0.2 µm filter, which allows the GMMA to pass through but which does not allow intact bacteria to pass through, or by using low speed centrifugation to pellet cells while leaving GMMA in suspension. Suitable purification methods are known in the art. A preferred two-step filtration purification process is described in WO2011/036562 herein incorporated by reference. Particularly the two-step filtration process is used to separate GMMA from cell culture biomass without using centrifugation.

GMMA containing compositions of the invention will generally be substantially free from whole bacteria, whether living or dead. The size of the GMMA means that they can readily be separated from whole bacteria by filtration e.g. as typically used for filter sterilisation. Although GMMA will pass through a standard 0.22µm filters, these can rapidly become clogged by other material, and so it may be useful to perform sequential steps of filter sterilisation through a series of filters of decreasing pore size before using a 0.22µm filter. Examples of preceding filters would be those with pore size of 0.8µm, 0.45µm, etc. GMMA are spontaneously-released from bacteria and separation from the culture medium, for example, using filtration, is convenient. Outer membrane vesicles formed by methods which involve deliberate disruption of the outer membrane (e.g. by detergent treatment, such as deoxycholate-extraction, or sonication) to cause outer membrane vesicles to form are excluded from the scope of the invention. GMMA used in the invention are substantially free from inner membrane and cytoplasmic contamination and contain lipids and proteins.

*Shigella* strains for use in the invention include one or more further changes relative to a wild-type strain. Particularly, strains for use with the invention include one or more mutations resulting in inactivation of *htrB, msbB1* and/or *msbB2.* By way of non-limiting example, suitable mutations may be selected from the group consisting of *ΔhtrB, ΔmsbB1* and Δ*msbB2*

Alternatively or additionally, the immune response is an immune activating response. As used herein "immune activating response" includes or means an immune response that increases inflammation, antibody-directed cell death and/or dormancy, and/or complement-mediated cell death and/or dormancy.

Alternatively or additionally, the immune response is antibody-directed. As used herein "antibody-directed" includes or means the induction of cell death and/or dormancy by an antibody-dependent mechanism.

Alternatively or additionally, the immune response comprises or consists of a protective immune response, e.g., an *in vitro* protective immune response and/or an *in vivo* protective immune response.

Alternatively or additionally, the immune response comprises or consists of complement-mediated killing.

As used herein, "complement-mediated killing" includes or means the induction of cell death and/or dormancy by a complement-dependent mechanism. Complement-mediated killing can be measured by any suitable means know to the skilled person, in particular, serum bactericidal assay (SBA) as described in the Examples section below.

Alternatively or additionally, the immune response comprises or consists of prevention or reduction of entry of *Shigella flexneri* cells into host macrophages and/or epithelial cells.

Measurement of *S*. *flexneri* interaction with and/or entry into host macrophages and/or epithelial cells can be determined using any suitable means known in the art, such as the methods taught in Raygoza-Anaya et al., 1990 'In vitro model for the analysis of the interaction between Shigella flexneri and the intestinal epithelium' Arch. Invest. Med. (Mex), 21(4):305-9; Willer Eda et al., 2004, 'In vitro adhesion and invasion inhibition of Shigella dysenteriae, Shigella flexneri and Shigella sonnei clinical strains by human milk proteins' BMC Microbiol., 28;4:18; Guhathakurta et al., 1999, 'Adhesion and invasion of a mutant Shigella flexneri to an eukaryotic cell line in absence of the 220-kb virulence plasmid' FEMS Microbiol. Lett., 181(2):267-75; or Bando et al., 2010, 'Expression of bacterial virulence factors and cytokines during in vitro macrophage infection by enteroinvasive Escherichia coli and Shigella flexneri: a comparative study' Mem. Inst. Oswaldo Cruz., 105(6):786-91, which are each incorporated by reference herein.

Since this organism is unable to invade epithelial cells through the apical route, Shigella exploits M cells, the specialized epithelial cells in the follicular associated epithelium (FAE) that overlie lymphoid tissue, to gain entry into the colonic epithelium (Wassef et al. 1989). M cells allow intact Shigella to traverse into the underlying subepithelial pocket where macrophages reside. Macrophages engulf Shigella, but instead of successfully destroying the bacteria in the phagosome, the macro- phage succumbs to apoptotic death (Zychlinsky et al. 1992). Prior to cell death, infected macrophages release IL-1b through the direct activation of caspase-1 by Shigella (Zychlinsky et al. 1994). The pro-inflammatory nature of this cytokine results in the recruitment of polymorphonuclear cells (PMNs) that infiltrate the infected site and destabilize the epithelium (Perdomo et al. 1994a,b). Loss of integrity of the epithelial barrier allows more bacteria to traverse into subepithelial space and gives these organisms access to the basolateral pole of the epithelial cells (Mounier et al. 1992). Shigella can then invade the epithelial cells lining the colon, spread from cell to cell and disseminate throughout the tissue. Cytokines released by infected epithelial cells attract increased numbers of immune cells to the infected site, thus compounding and exacerbating the inflammation.

Shigellosis produces a spectrum of clinical outcomes ranging from watery diarrhoea to classic dysentery characterized by fever, violent intestinal cramps and discharge of mucopurulent and bloody stools. Inflammation of the infected tissue is a key feature of shigellosis. Histopathological studies of colonic biopsies from infected patients reveal inflammatory cell infiltration into the epithelial layer, tissue oedema and eroded regions of the colonic epithelium (Mathan & Mathan 1991).

Alternatively or additionally, the immune response prevents, abolishes or reduces one or more symptom of *Shigella flexneri* infection selected from the group consisting of:
a. watery diarrhoea;
b. fever;
c. intestinal cramps;
d. abdominal pain;
e. tenesmus;
f. mucopurulent stools;
g. bloody stools;
h. inflammation of infected tissue (e.g., colon tissue [e.g., inflammatory cell infiltration into the epithelial layer]);
i. oedema of infected tissue (e.g., colon tissue);
j. faecal haemoglobin;
k. bacterial shedding;
l. erosion of colonic epithelium (number of eroded regions, diameter, depth); and
m. macrophage apoptotic cell death.

By "prevents, abolishes or reduces" we include or mean reduction in the symptom by at least 25%, at least 50%, at least 75%, at least 85%, at least 90%, at least 95%, at least 98% at least 99%, or at least 100%. Alternatively or additionally, the one or more symptom is reduced by at least 10%, for example, reduced by at least 15%, 20%, 25%, 30%, 35%, 40%, 45%, 50 %, 55%, 60%, 65%, 70%, 75%, 80%, 85%, 90%, 95%, 96%, 97%, 98%, 99% or 100%.

By "raising an immune response" we mean or include that the immune system is activated in a host following exposure to an antigen (e.g., the *Shigella flexneri* O-antigen).

Alternatively or additionally, the immune response is raised in a mammal.

Alternatively or additionally, the mammal is selected from the group consisting of armadillo *(dasypus novemcinctus),* baboon *(papio anubis; papio cynocephalus);* camel *(camelus bactrianus, camelus dromedarius, camelus ferus)* cat *(felis catus),* dog *(canis lupus familiaris),* horse *(equusferus caballus),* ferret *(mustela putorius furo),* goat *(capra aegagrus hircus),* guinea pig *(cavia porcellus),* golden hamster *(mesocricetus auratus),* kangaroo *(macropus rufus),* llama *(lama glama),* mouse *(mus musculus),* pig *(sus scrofa domesticus),* rabbit *(oryctolagus cuniculus),* rat *(rattus norvegicus),* rhesus macaque *(macaca mulatta),* sheep *(ovis aries)* and human *(Homo sapiens).*

Alternatively or additionally, the protective immune response is protective against a disease or condition caused by an organism selected from the group consisting of: *Shigella sonnei, Shigella flexneri, Shigella boydii,* and *Shigella dysenteriae.*

The terms "OMV" and "GMMA" may be used interchangeably herein.

A second aspect provides a binding moiety capable of specifically binding to one or more O-antigen defined in the first aspect.

By "specifically binding" we mean or include that the binding moiety binds at least 10-fold more strongly to its target antigen or epitope than to any other antigen or epitope (in particular, any other *Shigella* [in particular, *S*. *flexneri*] O-antigen or fragment thereof); preferably at least 50-fold more strongly and more preferably at least 100-fold more strongly. Preferably, the binding moiety of the invention specifically binds to the antigen or epitope under physiological conditions (for example, *in vivo;* and for example, during *S*. *flexneri* infection). Binding strength can be measured by surface plasmon resonance analysis using, for example, a BiacoreTM surface plasmon resonance system and BiacoreTM kinetic evaluation software (e.g., version 2.1).

Alternatively or additionally, the binding moiety is selected from the group consisting of: antibodies; antigen-binding fragments; and antibody mimetics. Alternatively or additionally, the binding moiety is an antibody. Alternatively or additionally, the antibody is polyclonal or monoclonal. Alternatively or additionally, the binding moiety is an antigen-binding fragment selected from the group consisting of: Fab (fragment antigen binding); F(ab')₂; Fab'; scFv (single chain variable fragment); di-scFv; sdAb (single domain antibody / domain antibody); trifunctional antibody; chemically-linked F(ab')₂; and BiTE (bi-specific T-cell engager). Alternatively or additionally, the antibody or antigen binding fragment thereof is an antigen binding fragment selected from the group consisting of affibodies molecules; affilins; affimers; affitins; alphabodies; anticalins; avimers; DARPins; fynomers; kunitz domain peptides; monobodies and nanoCLAMPs.

A third aspect provides a pharmaceutical composition comprising an O-antigen for use defined in the first aspect and/or a binding moiety as defined in the second aspect. Alternatively or additionally, the composition comprises an adjuvant. Yet more particularly, the adjuvant is an adsorbent. Still yet more particularly, the adjuvant is an adsorbent that does not enhance immunogenicity of GMMA, for example, as measured by anti- LPS antibody response. Particular adjuvants include, for example, aluminium adjuvants including aluminium hydroxide, ALHYDROGEL®, aluminium phosphate, potassium aluminium sulphate and alum.

A fourth aspect provides a kit comprising or consisting of an O-antigen for use defined in the first aspect, a binding moiety as defined in the second aspect and/or a pharmaceutical composition as defined in the third aspect; and (optionally) instructions for use.

A fifth aspect provides an O-antigen for use defined in the first aspect, a binding moiety as defined in the second aspect, a pharmaceutical composition as defined in the third aspect and/or a kit as defined in the fourth aspect, for use in medicine.

A sixth aspect provides an O-antigen for use defined in the first aspect, a binding moiety as defined in the second aspect, a pharmaceutical composition as defined in the third aspect and/or a kit as defined in the fourth aspect, for use in preventing or treating bacterial infection and/or symptoms thereof.

Alternatively or additionally, the bacterial infection is, wholly or in part, infection with one or more bacterium defined in the first aspect.

A seventh aspect provides an effective amount of an O-antigen in the first aspect, a binding moiety as defined in the second aspect, a pharmaceutical composition as defined in the third aspect and/or a kit as defined in the fourth aspect for use in the manufacture of a medicament for treating for the prevention or treatment of bacterial infection and/or symptoms thereof (e.g., where the bacterial infection is, wholly or in part, infection with one or more bacterium defined in the first aspect).

An eighth aspect provides a method of treating or preventing bacterial infection and/or symptoms thereof comprising administering a suitable amount of an O-antigen for use defined in the first aspect, a binding moiety as defined in the second aspect, a pharmaceutical composition as defined in the third aspect and/or a kit as defined in the fourth aspect.

A ninth aspect provides a binding moiety as defined in the second aspect for detecting the presence of bacteria, for example, wherein the bacteria are one or more bacterium defined in the first aspect. Alternatively or additionally, the detection is *in vitro* and/or *in vivo.*

A tenth aspect provides an O-antigen, binding moiety, pharmaceutical composition, kit, use or method as described in specification and figures herein.

### EXAMPLES

### 1. Introduction

A broadly-protective vaccine against shigellosis needs to cover multiple *S*. *flexneri* serotypes. A challenge is to design a practical vaccine that balances coverage versus complexity and cost. Importantly, we found, based on immunogenicity in mice, that a simple three-component vaccine of GMMA from *S*. *sonnei, S*. *flexneri* 1b and 3a would induce killing of most epidemiologically significant *Shigella* strains. This was not predicted based on cross-reactivity of currently described shared serotypes and serogroups. We don't know how these results translate to human immunogenicity - there are data that show humans recognized some *Shigella* serospecificities differently to mice. However, the study presented here provides a framework for empirically designing such a vaccine based on upcoming human vaccine trials.

### 2. Materials and Methods

### 2.1 Shigella Strains

*S*. *sonnei* 53G (32) was obtained from Walter Reed Army Institute of Research, Washington, D.C., USA. The *S. sonnei ΔvirG::*cat strain used in FACS and SBA was generated by Caboni *et al.* (33) to ensure a stable expression of OAg during growth by stabilization of the pSS virulence plasmid that contains the OAg cluster genes by culturing the bacteria in presence of chloramphenicol.

*S*. *flexneri* lines of the 14 subtypes were purchased from the Public Health England, London, UK. Working cell banks were prepared and typed using both agglutination and surface staining by FACS typing with the commercial *Shigella* typing antisera from Denka Seiken Co., Ltd; the type specific serum I, II, III, IV, V and grouping sera 3,4; 6; 7,8. Manufacturer's recommendations were followed for the agglutination. For FACS typing, bacteria were grown in LB medium, diluted to 2x10⁷ CFU/mL in PBS, then 50 µL were transferred in 96 well plate on ice, incubated with 1:400 dilution of typing and grouping antisera from Denka Seiken Co. Ltd., washed, then incubated with 1:1,000 dilution of fluorescein-conjugated F(ab')2 fragment goat anti-rabbit IgG specific (Jackson Immuno Research Europe Ltd.). The cells were then fixed for 3 h with BD Cytofix® (containing 4.2% formaldehyde), washed and then resuspended in 130 µl PBS. Samples were measured with a BD FACS Canto equipped with a high throughput sample reader using BD FACS DIVA version 8.0.1 software. Cells were gated on FSC-A versus SSC-A. The signal was then measured (FITC/fluorescein channel). Analyses were performed with FlowJo version 10.3 (FlowJo, LLC, Ashland, Oregon). The Mean Fluorescence Intensity (MFI) was used as the measure of strength of the staining. All lines gave the expected typing pattern. For *S*. *flexneri* X the reaction with group 7,8 antisera was weak; this weak reaction was not confirmed in the clone selected for GMMA production. By FACS analysis, an instability of the *S*. *flexneri* 5b cell line was identified; the population had a mixture of cells that were positive or negative for group 7,8 and thus a mixed *S*. *flexneri* 5a/5b phenotype, presumably due to variable expression of the *gtrX* gene encoding the glycosyl-transferase that distinguishes *S*. *flexneri* 5a from 5b. This was also true of the GMMA producing line derived from this line and thus the GMMA used for vaccination were probably a mixture of *S*. *flexneri* 5a and 5b. For use in the FACS and SBA assays, a new working cell line was selected from the *S*. *flexneri* 5b bacterial cells that uniformly reacted strongly with the group 7,8 antisera.

In addition to the serological typing, the lines used for the GMMA production and the target panel were genotyped by PCR for the genes that encode the group specific 9 (*oacB* or *oacC*) and 10 (*oacD*) phenotypes. The PCR reaction mixtures contained 12.5 µL DreamTaq Green PCR Master Mix (2x), 9.5 µL sterile water, 1 µL 10 mM forward primer, 1 µL 10 mM reverse primer and 1 µL template (bacteria suspended in water to an OD600 of 5). After amplification, the presence of the amplified gene was detected following electrophoresis on Ethidium bromide stained agarose gels.

### 2.2. GMMA production, purification and formulation

To generate the GMMA producing lines, the *tolR* gene was deleted as described for the generation of the *S*. *sonnei ΔtolR* mutant (34). The resulting clonal lines were re-typed to assure that the cloning process had not changed serotype and serogroup specificities.

These GMMA were used to immunize mice, but the resulting sera did not react with OAg positive homologous bacteria and the results are not included in this study. As for the parent line, most, but not all, of the *S*. *flexneri* 5b GMMA producing bacteria typed by FACS as *S*. *flexneri* 5a (i.e. negative for group 7,8). These GMMA were used to immunize mice and the resulting sera were included in the cross-reaction panel testing. Bacterial strains were grown at 30°C on LB agar or in liquid chemically defined medium (SDM), as described (34, 35). When required, kanamycin (30 µg/mL), was added for selection of the GMMA producing strains. For GMMA production, overnight cultures were used to inoculate the SDM at an OD₆₀₀ of 0.03-0.05 and incubated at 30 °C and 200 rpm to an OD₆₀₀ 8-10. Culture supernatants were collected by centrifugation followed by a 0.22-µm filtration, ultracentrifuged and the resulting pellet containing GMMA resuspended in PBS as described (35).

GMMA quantities were expressed as total protein present using the micro-BCA protein assay (Bio-Rad) kit according to the manufacturer's instructions, using Bovine serum albumin (Pierce) for the standard curve. The amount of OAg in the GMMA was determined by HPAEC-PAD analysis by measuring rhamnose content, (3 rhamnose residues per repeating units (RU) for all *S*. *flexneri* serotypes except *S*. *flexneri* 6 for which there are 2). The OAg to protein ratio in the GMMA varied from 0.39 to 0.8 *(SI Appendix,* Table S2). GMMA from *S*. *flexneri* X contained lower amount of OAg (the OAg /protein ratio was 0.12 for *S*. *flexneri* X).

The GMMA were adsorbed onto aluminum hydroxide (Alhydrogel 2%, Brenntag Biosector, Denmark). GMMA were added to Alhydrogel to give 4 µg/mL GMMA protein and 0.7 mg Al3+/mL in 10 mM Tris, pH 7.4 and 9 g/L NaCl, then stirred for 2h. Preparations were tested to show they had no bacterial contamination and were stored at 2-8 °C for one week prior to use.

### 2.3. Immunogenicity studies in mice

Animal studies were performed as part of the Italian Ministry of Health Animal Ethics Committee project number 201309. Four CD1 mice per group (female, 4 to 6 weeks old) were immunized intraperitoneally (500 µL each mouse) with 2 µg of GMMA (protein) on days 0 and 21; sera were collected on day 21 and 35 (bleed out). The day 35 sera were pooled and used for the studies reported in this paper.

### 2.4. Cross-reactivity measured by FACS

Prior to assessment of cross-reactivity, all the *S*. *flexneri* bacteria from the different serotypes used in the study were tested for binding of sera raised against OAg negative *S.flexneri* 2a GMMA using the methodology described below.

Surface staining of the panel of 11 OAg positive *S*. *flexneri* lines was carried out with the pooled day 35 sera from the 14 immunization groups and pooled sera similarly raised against an OAg negative *S*. *flexneri* 2a GMMA. The sera were also tested on OAg positive and negative *S*. *sonnei* and the sera raised against OAg negative *S*. *flexneri* 2a were also tested on OAg negative *S*. *flexneri* 2a bacteria.

The pooled day 35 sera, were added to the bacterial suspensions, incubated for 1 h, washed, then APC-conjugated anti-mouse IgG (1:400 dilution) was added and incubated for 1 h. The signal was then measured in the APC channel. The baseline was set by *S*. *flexneri* 1b, 2a, 3a and 6 controls incubated only with the secondary antibodies and without any mouse serum. A matrix showing the mean fluorescence intensities (MFI) of surface staining of *S*. *flexneri* wild type bacteria lines of the different serotypes is reported in Supplementary information, Table S3.

### 2.5. High through-put Luminescence - Serum bactericidal assay (L-SBA)

SBA were performed as described (36). Briefly, *S*. *sonnei* and *S*. *flexneri* bacteria derived from the same working cell banks used for the FACS were grown to log-phase (OD: 0.2), diluted 1:1,000 in PBS and distributed in 96-well plates. To each well, dilutions of heat-inactivated pooled mouse sera and active Baby Rabbit Complement (BRC; 7-20% of the final volume) was added. As control, bacteria were incubated with sera plus heat-inactivated BRC, sera alone (no BRC), SBA buffer or active BRC. After 3h incubation, surviving bacteria were determined by measuring ATP. SBA is reported in serum titers, defined as serum dilutions giving 50% inhibition of the ATP level in the positive control. Titers below the minimum measurable titer of 100 was assigned titer of 10. A matrix showing serum titers on *S*. *flexneri* wild type cell lines of the different serotypes is reported in Supplementary information, Table S4.

### 2.6. Modelled SBA heat map

The observed average log (SBA titer) for sera tested on the homologous serotypes (i.e. anti-S. *flexneri* 2a antisera tested on *S*. *flexneri* 2a or on *S*. *flexneri* 2b) was 4.7. Therefore, in constructing a theoretical SBA heat map, the SBA log titer) for sera tested on homologous serotypes was assigned a value of 4.7. The observed average SBA log titer tested on heterologous serotypes where the SBA was measurable was 3.9. Where a vaccinating GMMA shared a single strongly typing group specificity we assigned a value of 3.9 to this interaction. As shown in Supplementary Information *(SI Appendix,* Table S1), typing of the target bacteria with the standard group-specific reagents showed several strains that gave positive but weak interaction with typing reagents. On average these had log MFI that were 0.9 (group 3,4) or 0.7 (group 7,8) log units lower than the high responders. In this case we assigned a value of 3.1 (i.e. 0.8 log units lower than the high responders) to the modelled SBA value (we assumed that a weakly typing positive GMMA producing strain still had sufficient group specific antigen to elicit a full group specific antibody response). Where the immunizing GMMA and the target bacteria shared two group specificities we assigned an SBA log titer as the log of the sum of the titers. Thus, the modelled titer of anti-S. *flexneri* 1a GMMA on *S*. *flexneri* 2a that share both the 3,4 and the 9 group specificities is assigned an SBA log titer) of 4.2 = log (10^3.9 + 10^3.9). For both the observed SBA titers and the modelled SBA titers, a calculated SBA log titer that could be obtained by immunizing with a mixture of *S*. *flexneri* 1a and 3a was calculated similarly: E.g. the estimated SBA log titer of a mixture of anti-*S*. *flexneri* 1b and 3a GMMA on *S*. *flexneri* 2a was 4.0 = log (10^3.9 + 10^3.1).

### 3. Results

### 3.1. Serotype and group specificities of the bacteria used in this study

A summary of the serotype and group specificities of the bacteria used in this study based on typing with specific antisera or inferred by the presence of genes encoding O-acetylases are shown in Table 1. The presence of *O-*acetylation was demonstrated by NMR for *S*. *flexneri* 1b, 2a and 3a. The details of the typing are included in the Supplementary Information *(SI Appendix,* Table S1).

### 3.2. Evaluation of cross-reactivity and cross functionality of antibodies raised in mice against GMMA from one subtype of S. flexneri on heterologous S. flexneri subtypes

### 3.2.1. Evaluation of cross-reactivity by FA CS

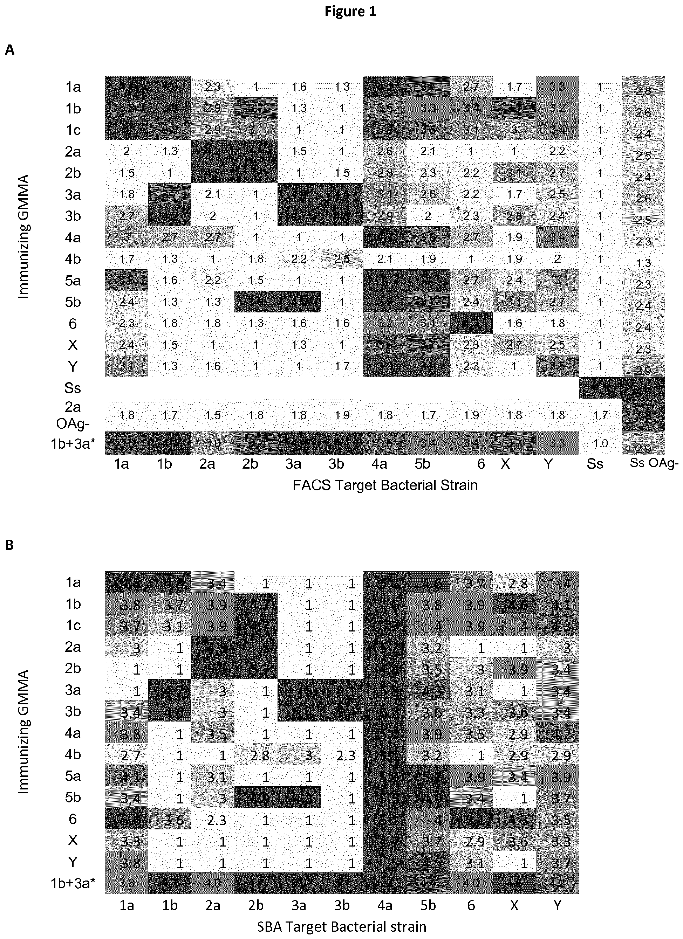

A heat map was generated with the Log₁₀ of the Mean Fluorescence Intensities (Log MFI) of surface staining of a panel *S*. *flexneri* bacteria to visualize the cross-reactivity patterns (Fig. 1A). The detailed MFI values are reported in the Supplementary Information *(SI Appendix,* Table S3). A threshold criterion was applied to distinguish relevant cross-reactivity: a level of cross-reactivity that can be predictive of field cross-coverage from low level cross-reactivity unlikely to provide field cross-coverage. This threshold was estimated based on literature evidence that in preclinical animal models, there is an inability to protect against challenge from *S*. *flexneri* 3a animals immunized with *S*. *flexneri* 2a and vice-versa (13, 20). For FACS experiment this threshold was estimated to be MFI ≥ 130. By the assessment of the staining intensity of the antibodies raised by the vaccinating serotype on homologous and heterologous binding serotypes it was possible to identify broad specificity immunogens.

*Binding of sera raised against OAg negative GMMA:* Antisera raised against OAg negative *S*. *flexneri* 2a GMMA (GMMA from *S*. *flexneri* 2a *ΔtolR ΔrfbG)* gave strong fluorescence on OAg negative *S*. *flexneri* 2a bacteria (MFI 5000) and OAg negative *S*. *sonnei* (MFI 6300); binding was undetectable on all tested OAg positive bacteria, including OAg positive *S*. *flexneri* 2a.

### 3.2.2. Binding of sera raised against O Antigen positive GMMA

### 3.2.2.1. Binding to O antigen negative S. sonnei

All the antisera raised with OAg positive GMMA gave detectable binding to OAg negative *S*. *sonnei.* Anti-*S*. *flexneri* 4b had the weakest binding (MFI 40). All including anti-S. *flexneri* 4b, gave an MFI that was more intense on the *S*. *sonnei* OAg negative GMMA than to at least one of the OAg positive *S*. *flexneri* tested.

### 3.2.2.2. Homologous binding (binding to the parent bacteria of the immunizing GMMA)

All homologous sera gave strong binding, ranging from MFI of 4,508 (Log MFI 3.7) for *S*. *flexneri* 5b to 98,520 (Log MFI 5.0) for *S*. *flexneri* 2b, except for *S*. *flexneri* X that gave relatively weak binding to *S*. *flexneri* X bacteria (MFI 541, log MFI 2.7). *S*. *flexneri* 4b pooled serum gave generally weak binding but was not tested for binding to the parent *S*. *flexneri* 4b.

### 3.2.2.3. Heterologous binding (binding to bacteria not the parent of the immunizing GMMA)

For most of the antisera tested, the highest level of cross-reaction was identified among homologous serotypes (*S*. *flexneri* serotypes having a common glucosyl or acetyl modification at the same position on the OAg backbone e.g. *S*. *flexneri* 1c antisera binding to *S*. *flexneri* 1a and 1b bacteria). The level of cross-reactivity varied: antisera from *S*. *flexneri* 2a GMMA strongly reacted only with the homologous serotypes and only weakly with two other serotypes *S*. *flexneri* 4a and Y (i.e. with an MFI > 130 for 2/9 heterologous serotypes tested). By contrast, antisera against *S*. *flexneri* 1b GMMA elicited broad cross-reactions to homologous serotypes and most heterologous serotypes giving an MFI >130 to 7/9subtypes from heterologous serotypes. Thus *S*. *flexneri* 1b, 1c, 3b, 4a, 5a and 5b GMMA are broad-specificity immunogens by FACS (MFI > 130 on ≥ 60% heterologous serotypes/subtypes); *S*. *flexneri* 1a, 2b, 3a and X, medium-specificity immunogens (MFI>130 on 50% to < 60% heterologous serotypes/subtypes) and *S*. *flexneri* 2a, 6 and Y, narrow-specificity immunogens (MFI > 130 on <50% heterologous serotypes/subtypes). *S*. *flexneri* 4b GMMA had an indeterminate breadth of specificity. As the 4b GMMA failed to generate strong binding to homologous serotypes (i.e. *S*. *flexneri* 4a) and to OAg negative bacteria, the lack of binding to other serotypes may only be indicative of a poor immunogenicity of these GMMA.

The subtypes varied considerably in their ability to be recognized by heterologous sera. Some of the subtypes were widely recognized by many different antisera, specifically *S*. *flexneri* 1a, 4a, 5b, 6, X and Y. Thus, these are broad-specificity targets. By contrast, some subtypes were only recognized by a few antisera. *S*. *flexneri* 3b was the most restricted target, only recognized strongly by sera raised against *S*. *flexneri* 3a or 3b and weakly by sera raised against *S*. *flexneri* 4b. *S*. *flexneri* 3a was the next most restrictedly recognized subtype with binding only by anti-*S*. *flexneri* 3b and 5b antisera. By these criteria, *S*. *flexneri* 1b, 2a, 2b, 3a and 3b are narrow-specificity targets.

As expected, *S*. *sonnei* bacteria were not stained by any of the *S*. *flexneri* GMMA antisera.

### 3.3. Evaluation of cross-functionality by Serum Bactericidal Activity (SBA)

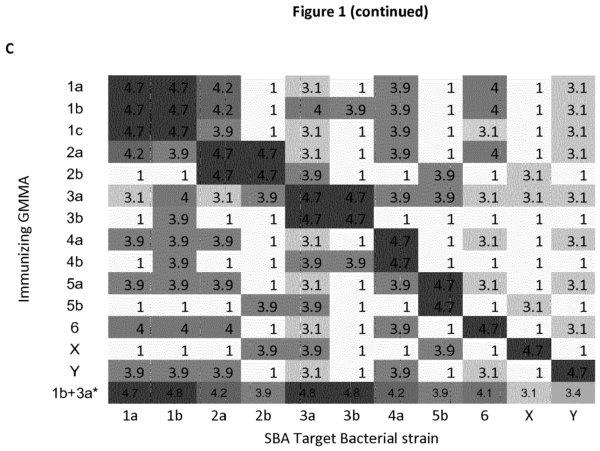

A heat map of SBA data containing the Log₁₀ IC50 of the pooled sera on *S*. *flexneri* bacterial cell lines is shown in Fig. 1*B*. The detailed IC50 titers are reported in the supplementary information *(SI Appendix,* Table S4) As for FACS, a threshold criterion was applied to distinguish relevant cross-reactivity. This threshold was estimated as an IC₅₀ > 500 (this threshold was the cut-off for the absence of killing of the control GMMA from *S*. *flexneri* 2a By assessment of the antisera killing capabilities of vaccinating GMMA serotype it was possible to identify broad-specificity immunogens.

The binding of antibodies judged by FACS and killing as judged by SBA was similar (Fig. 1A compared to Fig. 1*B*). There were few inconsistencies between the FACS and SBA data. Antisera against *S*. *flexneri* 4a and 5b gave moderate intensity MFI on *S*. *flexneri* 1b and X respectively, but failed to give detectable SBA titers. *S*. *flexneri* 3b, 4b and 6 GMMA gave relatively stronger SBA titers on more targets than expected from the FACS data. The breadth of the specificity was a little broader as judged by SBA than FACS. Thus the *S*. *flexneri* 1b, 1c, 3b, 4a, 5a and 5b serotypes GMMA, identified as broad-specificity immunogens by FACS, were joined by *S*. *flexneri* 3a 5a and 6 (IC50 ≥1,000 on ≥ 60% heterologous serotypes). There were fewer medium-medium specificity immunogens (*S.flexneri* 1a and 2b) and *S*. *flexneri* 2a joining *S. flexneri* 4b, X, Y as narrow-specificity immunogens.

On the other hand, the heat map of SBA data poorly correlated with a heat map predicted from the reactivity expected from serotype and group antigens (Fig. 1C).

### 4. Discussion

There are only a few reports of cross-reactivity among *S*. *flexneri* serotypes and subtypes in the literature. An extensive screening using preclinical animal models to identify cross-reactive antibodies and the structural basis of cross-reactivity has not been carried out.

In this study we used FACS and SBA, the two techniques that give a direct measure of interactions between host antibody response and infective bacteria. The SBA assay is the method of choice to evaluate the complement-mediated functional activity of antibodies induced by a bacterium during infection; additionally, for *Neisseria meningitidis,* SBA is the accepted correlate of protection on which this vaccine is registered.

GMMA contain all the outer membrane components of their parent bacteria (19) and thus could elicit antibodies that bind to many bacterial surface components. Indeed, as measured by FACS, OAg negative GMMA (i.e. *S*. *flexneri* 2a *ΔtolR ΔrfbG* GMMA) elicit antibodies that strongly bind to bacteria without OAg, suggesting that the GMMA can induce a broad range of antibody responses. However, three observations from this study show that the antibody induced by OAg positive GMMA measured by FACS and by SBA on OAg positive bacteria are dominantly directed against the OAg (Fig. 2).
1. The observed FACS and SBA responses are predominantly serotype or subtype specific and no *S*. *flexneri* GMMA induced immune responses that recognized *S*. *sonnei* which has a similar LPS core oligosaccharide (21) and most of the outer membrane proteins (19). Furthermore, for each pool of anti-S. *flexneri* antisera there was at least one subtype of *S*. *flexneri* bacteria to which the antisera failed to give a binding stronger than that seen on *S*. *sonnei,* again despite sharing most outer membrane components other than the OAg. The negative subtypes differed depending on the specificity of the pool. E.g. anti-S. *flexneri* 1a gave no detective binding to *S*. *flexneri* 3a (Fig. 1A and 1*B*)
2. Sera raised against GMMA from OAg negative bacteria had no or very weak binding detectable to OAg positive bacteria but very strong binding to OAg negative bacteria.
3. Sera raised against OAg positive GMMA from *S*. *flexneri* or *S*. *sonnei* GMMA bound to OAg negative *S*. *sonnei.*

Although all the sera have antibodies capable of significant binding to the surface of bacteria, they are unable to do so if the bacteria have an OAg coat (Fig. 2). This is in agreement with earlier findings from immunization studies with intact bacteria (22) suggesting that the OAg shields the bacteria from binding to antigen on the surface of the outer membrane and that the observed binding must be to dominant surface components that do differ from one serotype to another - i.e. the OAg.

There are two important consequences for antibodies generated by GMMA:
- The observed strain specificity and cross-reactivity must predominantly be directed against epitopes in the OAg of each serotype.
- The OAg specificities induced by GMMA will be important for inducing broad protection from a vaccine by binding of antibody to the surface of bacteria.

This is consistent with the observation that the immunity in human elicited by attenuated *Shigella* strains is dominantly OAg specific and with the results of earlier animal studies with immunization by killed or attenuated bacteria (23-25). Given the complex mechanism by which *Shigella* invades the intestinal lumen and the infection is established, this does not rule out protection via other mechanism not involving OAg, e.g. T cell response against macrophages or other cells containing intracellular *Shigella* (26-29) .

The data from both FACS and SBA showed that, as expected, the different GMMA generated substantial cross-reactivity on strains of *S*. *flexneri* that shared the same serotype specificities. For example, antisera to *S*. *flexneri* 2a GMMA bound strongly to *S*. *flexneri* 2b bacteria and *vice versa.* These two serotypes only share the Type II epitopes and no group specificities. Importantly there was also substantial binding to strains that did not share the same type specificities. For example, antisera to *S*. *flexneri* 1a, 1b and 1c GMMA bound strongly to *S*. *flexneri* 2a bacteria.

It has been generally assumed that for immunizing and target pairs that do not share the same type specificity, cross-reactivity will be mediated by the group specificities (i.e. epitopes 3,4; 6; 7,8; 9 and 10). This was the basis of the experimental cross-protecting vaccine developed by Noriega et *al.,* (13) based on attenuated *S*. *flexneri* 2a and *S*. *flexneri* 3a to deliver Type II and III and group 3,4; 6 and 7,8 specificities.

However, the pattern of cross-reactivity observed with the larger panel in this GMMA study was unexpected: detailed comparison of the cross-protection modelled on shared group specificities (Fig. 1*B* compared to Fig. 1C) has little resemblance to what was observed. The modelling is not sensitive to the detailed assumptions used to create Fig. 1C. Just scoring the reaction as being detectable or not detectable gives a similar picture. For example, antisera raised against *S*. *flexneri* 1b and 1c GMMA gave some of the strongest binding observed to *S*. *flexneri* 2b, which share no known group specificities. There are also multiple cases where cross-reaction was expected from shared group specificities, but not observed. For example, antisera raised against *S*. *flexneri* Y GMMA did react with *S*. *flexneri* 1a bacteria as expected with uniquely shared 3,4 specificity, but gave no detectable binding to *S*. *flexneri* 1b and *S*. *flexneri* 2a, which strongly react with group 3,4 typing sera. Thus, even the observed cross-reaction between anti *S*. *flexneri* Y sera and *S*. *flexneri* 1a bacteria is almost certainly not due to group 3,4 reactivity.

Therefore, we conclude that most of the cross-reactivity cannot be explained by group specificities.

A feature was the lack of reciprocity between immunogen and antigen. For example, *S*. *flexneri* 3b GMMA generated substantial SBA titers and to a lesser extent FACS MFI against all 9 of the 10 non-homologous OAg positive *S*. *flexneri* strains tested. By contrast, other than a weak reactivity generated by *S*. *flexneri* 4b, the only other strain to generate detectable SBA/FACS activity against *S*. *flexneri* 3b, was *S*. *flexneri* 3a. Similarly, *S*. *flexneri* 1b GMMA generated substantial SBA/FACS activity against 8/10 heterologous *S*. *flexneri* OAg positive *S*. *flexneri* strains (except *S*. *flexneri* 3a and 3b). In fact, the cross-reactivity was so broad that a bivalent vaccine consisting only of *S*. *flexneri* 1b and 3a could give antibodies in the mouse that react strongly with all isolates tested (Fig. 1*A* and 1*B*)

The opposite was also observed. GMMA from *S*. *flexneri* 2a, 4b, X and Y and, to a lesser extent, *S*. *flexneri* 6 generated antibody that reacted with relatively few other isolates. All, except *S*. *flexneri* 4b, generated significant reaction by FACS to OAg negative *S*. *sonnei,* suggesting that they were intrinsically immunogenic, at least for non OAg components. In contrast to the poor immunogenicity observed, *S*. *flexneri* 2a, 4b, 5a, 6, X and Y were commonly recognized by antisera from other serotypes suggesting that inclusion of these serotypes in a vaccine would be less critical since there would be a high likelihood of being covered through cross-reactions.

Finding that the cross-reactivities do not match the known group specificities reflects older data on the generation of type and group specific typing sera. Initially sera raised against a strain of bacteria have extensive cross-reactions and it is only after exhaustive adsorption to remove the cross-reactions that the sera are useful as mono-specific typing reagents (30).

This lack of correlation of cross-reactivity and serotype/group specificities limits the rational design of combination vaccines based only on these serotype and group specificities. Despite that, the observation of extensive cross-reactivity in this mouse system and the observation of broadly specific immunogens such as *S*. *flexneri* 1b and 3a is encouraging, suggesting that practical *Shigella* vaccines may be possible that cover multiple serotypes with limited components due to currently undescribed specificities. There is an important caveat: these data are generated from mouse studies and there is at least one set of data from humans that show that the mouse results may not always be translatable to humans (31). As found in this study with mice immunized with *S*. *flexneri* 2a GMMA (Fig. 1), mice immunized with a *S. flexneri* 2a OAg conjugate also did not elicit antibody that reacted with *S*. *flexneri* 6 OAg although sera from people immunized with the *S.flexneri* 2a OAg conjugate did elicit antibody that bound to *S*. *flexneri* 6 OAg and may have protected children against infection with this strain (31). Clearly, careful analysis of the fine specificity of human sera coming from vaccines trials with *S*. *flexneri* constructs will be important for designing a broadly-specific *S*. *flexneri* vaccine.

### REFERENCES

1. Institute for Health Metrics and Evaluation (IHME) (2017) GBD Compare Data Visualization.
2. Kotloff KL, et al. (2013) Burden and aetiology of diarrhoeal disease in infants and young children in developing countries (the Global Enteric Multicenter Study, GEMS): a prospective, case-control study. Lancet 382(9888):209-222.
3. Pozzi C, Martin LB, & Saul A (2019) A systematic review of the distribution of serotypes causing Shigella disease in low and middle-income countries from 2006 to 2018 to inform vaccine development PLoS neglected tropical diseases.
4. Barry EM, et al. (2013) Progress and pitfalls in Shigella vaccine research. Nat Rev Gastroenterol Hepatol 10(4):245-255.
5. Camacho Al, Irache JM, & Gamazo C (2013) Recent progress towards development of a Shigella vaccine. Expert Rev Vaccines 12(1):43-55.
6. Robbins JB, Chu C, & Schneerson R (1992) Hypothesis for vaccine development: protective immunity to enteric diseases caused by nontyphoidal salmonellae and shigellae may be conferred by serum IgG antibodies to the O-specific polysaccharide of their lipopolysaccharides. Clin Infect Dis 15(2):346-361.
7. Liu B, et al. (2008) Structure and genetics of Shigella O antigens. FEMS Microbiol Rev 32(4):627-653.
8. Perepelov AV, et al. (2012) Shigella flexneri O-antigens revisited: final elucidation of the O-acetylation profiles and a survey of the O-antigen structure diversity. FEMS Immunol Med Microbiol 66(2):201-210.
9. Allison GE & Verma NK (2000) Serotype-converting bacteriophages and O-antigen modification in Shigella flexneri. Trends Microbiol 8(1):17-23.
10. Sun Q, et al. (2014) Serotype-converting bacteriophage Sfll encodes an acyltransferase protein that mediates 6-O-acetylation of GlcNAc in Shigella flexneri O-antigens, conferring on the host a novel O-antigen epitope. J Bacteriol 196(20):3656-3666.
11. Knirel YA, et al. (2015) O-antigen modifications providing antigenic diversity of Shigella flexneri and underlying genetic mechanisms. Biochemistry (Mosc) 80(7):901-914.
12. Pupo GM, Lan R, & Reeves PR (2000) Multiple independent origins of Shigella clones of Escherichia coli and convergent evolution of many of their characteristics. Proc Natl Acad Sci U S A 97(19):10567-10572.
13. Noriega FR, et al. (1999) Strategy for cross-protection among Shigella flexneri serotypes. Infect Immun 67(2):782-788.
14. Livio S, et al. (2014) Shigella isolates from the global enteric multicenter study inform vaccine development. Clin Infect Dis 59(7):933-941.
15. Gerke C, et al. (2015) Production of a Shigella sonnei Vaccine Based on Generalized Modules for Membrane Antigens (GMMA), 1790GAHB. PLoS One 10(8):e0134478.
16. Launay O, et al. (2017) Safety Profile and Immunologic Responses of a Novel Vaccine Against Shigella sonnei Administered Intramuscularly, Intradermally and Intranasally: Results From Two Parallel Randomized Phase 1 Clinical Studies in Healthy Adult Volunteers in Europe. EBioMedicine 22:164-172.
17. Launay O, et al. (2019) Booster Vaccination With GVGH Shigella sonnei 1790GAHB GMMA Vaccine Compared to Single Vaccination in Unvaccinated Healthy European Adults: Results From a Phase 1 Clinical Trial. Frontiers in immunology 10:335.
18. Obiero CW, et al. (2017) A Phase 2a Randomized Study to Evaluate the Safety and Immunogenicity of the 1790GAHB Generalized Modules for Membrane Antigen Vaccine against Shigella sonnei Administered Intramuscularly to Adults from a Shigellosis-Endemic Country. Frontiers in immunology 8:1884.
19. Maggiore L, et al. (2016) Quantitative proteomic analysis of Shigella flexneri and Shigella sonnei Generalized Modules for Membrane Antigens (GMMA) reveals highly pure preparations. Int J Med Microbiol 306(2):99-108.
20. Van De Verg LL, et al. (1996) Cross-reactivity of Shigella flexneri serotype 2a O antigen antibodies following immunization or infection. Vaccine 14(11):1062-1068.
21. Knirel YA, et al. (2011) Lipopolysaccharide core structures and their correlation with genetic groupings of Shigella strains. A novel core variant in Shigella boydii type 16. Glycobiology 21(10):1362-1372.
22. Kim MJ, et al. (2018) Cross-Protective Shigella Whole-Cell Vaccine With a Truncated O-Polysaccharide Chain. Front Microbiol 9:2609.
23. Kotloff KL, et al. (1996) Safety, immunogenicity, and transmissibility in humans of CVD 1203, a live oral Shigella flexneri 2a vaccine candidate attenuated by deletions in aroA and virG. Infect Immun 64(11):4542-4548.
24. Karnell A, Sweiha H, & Lindberg AA (1992) Auxotrophic live oral Shigella flexneri vaccine protects monkeys against challenge with S. flexneri of different serotypes. Vaccine 10(3):167-174.
25. Vecino WH, et al. (2004) Mucosal immunization with attenuated Shigella flexneri harboring an influenza hemagglutinin DNA vaccine protects mice against a lethal influenza challenge. Virology 325(2):192-199.
26. Sansonetti PJ, Arondel J, CanteyJR, Prevost MC, & Huerre M (1996) Infection of rabbit Peyer's patches by Shigella flexneri: effect of adhesive or invasive bacterial phenotypes on follicle-associated epithelium. Infect Immun 64(7):2752-2764.
27. Wassef JS, Keren DF, & Mailloux JL (1989) Role of M cells in initial antigen uptake and in ulcer formation in the rabbit intestinal loop model of shigellosis. Infect Immun 57(3):858-863.
28. Zhang Z, Jin L, Champion G, Seydel KB, & Stanley SL, Jr. (2001) Shigella infection in a SCID mouse-human intestinal xenograft model: role for neutrophils in containing bacterial dissemination in human intestine. Infect Immun 69(5):3240-3247.
29. Zychlinsky A, et al. (1996) In vivo apoptosis in Shigella flexneri infections. Infect Immun 64(12):5357-5365.
30. Gray SB, Jr., Green JH, Harrell WK, Britt L, & Bolin RC (1974) Reuse of Salmonella and Shigella absorbing cells for preparing monospecific Salmonella O and Shigella antisera. Appl Microbiol 28(2):320-322.
31. Farzam N, et al. (2017) Vaccination with Shigella flexneri 2a conjugate induces type 2a and cross-reactive type 6 antibodies in humans but not in mice. Vaccine 35(37):4990-4996.
32. Formal SB, et al. (1966) Protection of monkeys against experimental shigellosis with a living attenuated oral polyvalent dysentery vaccine. J Bacteriol 92(1):17-22.
33. Caboni M, et al. (2015) An O antigen capsule modulates bacterial pathogenesis in Shigella sonnei. PLoS Pathog 11(3):e1004749.
34. Berlanda Scorza F, et al. (2012) High yield production process for Shigella outer membrane particles. PLoS One 7(6):e35616.
35. Rossi O, et al. (2014) Modulation of endotoxicity of Shigella generalized modules for membrane antigens (GMMA) by genetic lipid A modifications: relative activation of TLR4 and TLR2 pathways in different mutants. J Biol Chem 289(36):24922-24935.
36. Necchi F, Saul A, & Rondini S (2018) Setup of luminescence-based serum bactericidal assay against Salmonella Paratyphi A. J Immunol Methods.
37. Ferrecio et al., 1991. Epidemiologic patterns of acute diarrhea and endemic Shigella infections in children in a poor periurban setting in Santiago, Chile. Am. J. Epidemiol. 134:614-627.
38. Mel et al., 1971. Studies on vaccination against bacillary dysentery 6. protection of children by oral immunization with streptomycin-dependent Shigella strains. Bull. WHO, 45:457.
39. Karnell et al., 1992. Auxotrophic live oral Shigeila flexneri vaccine protects monkeys against challenge with S. flexneri of different serotypes. Vaccine 1992, 10, 167.

### TABLES

**Table 1. Type and group specificities of the S. flexneri bacteria used in this study**

| | **Type Specificities** | | | | | | **Group Specificities***** | | | | |
|---|---|---|---|---|---|---|---|---|---|---|---|
| | **I** | **II** | **III** | **IV** | **V** | **VI** | **3,4** | **6** | **7,8** | **9** | **10** |
| **1a** | **+** | | | | | | **+** | | | **+** | |
| **1b** | **+** | | | | | | **+** | **+** | | **+** | |
| **1c*** | **+** | | | | | | **(+/-)^{†}** | | | | |
| **2a** | | **+** | | | | | **+** | | | **+** | **+** |
| **2b** | | **+** | | | | | | | **+** | | |
| **3a** | | | **+** | | | | **(+/-)** | **+** | **+** | | |
| **3b** | | | **+** | | | | | **+** | | | |
| **4a** | | | | **+** | | | **+** | | | | |
| **4b** | | | | **+** | | | | **+** | | | |
| **5a** | | | | | **+** | | **+** | | | | |
| **5b**** | | | | | **+** | | | | **+** | | |
| **6** | | | | | | **+** | **(+/-)** | | | **+** | |
| **X** | | | | | | | | | **(+/-)** | | |
| **Y** | | | | | | | **(+/-)** | | | | |
| **S. flexneri* 1c is also classified as *S. flexneri* 7a | | | | | | | | | | | |
| ***S. flexneri* 5b bacteria used in this study typed 5b. However, the *S. flexneri* 5b GMMA, by FACS had a mixed expression of group 7,8 and thus types as a mixture of *S*. *flexneri* 5a and 5b | | | | | | | | | | | |
| *** Groups 9 and 10 are defined by the PCR genotyping, not by phenotyping with type specific sera | | | | | | | | | | | |
| †Specificities that were positive by agglutination or by FACS but gave a titre approximately an order lower than other positive reactions. See Text and Supplementary Information Table S1 for details. | | | | | | | | | | | |

**Alternate Table 1. Type and group specificities of the S. flexneri bacteria used in this study**

| | **Type Specificities** | | | | | | **Group Specificities***** | | | | |
|---|---|---|---|---|---|---|---|---|---|---|---|
| | I | **II** | **III** | **IV** | **V** | **VI** | **3,4** | **6** | **7,8** | **9** | **10** |
| **1a** | 7,500 | 30 | 50 | 60 | 60 | 40 | 7,450 | 30 | 40 | **+** | |
| **1b** | 8,100 | 50 | 60 | 60 | 50 | 60 | 7,200 | 8,950 | | + | |
| **1c*** | 7,900 | 60 | 150 | 50 | 60 | 40 | | | | | |
| **2a** | 50 | 9,200 | 90 | 60 | 60 | 40 | 5,200 | 30 | 25 | + | + |
| **2b** | 70 | 8,700 | 60 | 60 | 30 | 40 | | | 1,530 | | |
| **3a** | 30 | 40 | 8,500 | 60 | 30 | 60 | 1,200 | 6,250 | 3,540 | | |
| **3b** | 50 | 90 | 7,200 | 50 | 40 | 80 | 30 | 4,500 | 45 | | |
| **4a** | 80 | 60 | 30 | 7,500 | 30 | 40 | 2,350 | 25 | 30 | | |
| **4b** | 90 | 80 | 60 | 7,600 | 30 | 60 | 20 | 1,250 | 30 | | |
| **5a** | 30 | 60 | 40 | 80 | 5,800 | 80 | 6,500 | 40 | 30 | | |
| **5b**** | 250 | 120 | 170 | 30 | 6,600 | 60 | 40 | 30 | 620 | | |
| **6** | 150 | 190 | 120 | 90 | 60 | 2,400 | 250 | 30 | 30 | + | |
| **X** | 80 | 90 | 110 | 60 | 90 | 60 | 20 | 30 | 450 | | |
| **Y** | 150 | 180 | 90 | 90 | 90 | 80 | 540 | 20 | 20 | | |
| **S. flexneri* 1c is also classified as *S. flexneri* 7a | | | | | | | | | | | |
| *** S. flexneri* 5b bacteria used in this study typed 5b. However, the *S. flexneri* 5b GMMA, by FACS had a mixed expression of group 7,8 and thus types as a mixture of *S*. *flexneri* 5a and 5b | | | | | | | | | | | |
| *** Groups 9 and 10 are defined by the PCR genotyping, not by phenotyping with type specific sera and thus are only typed as positive or negative | | | | | | | | | | | |

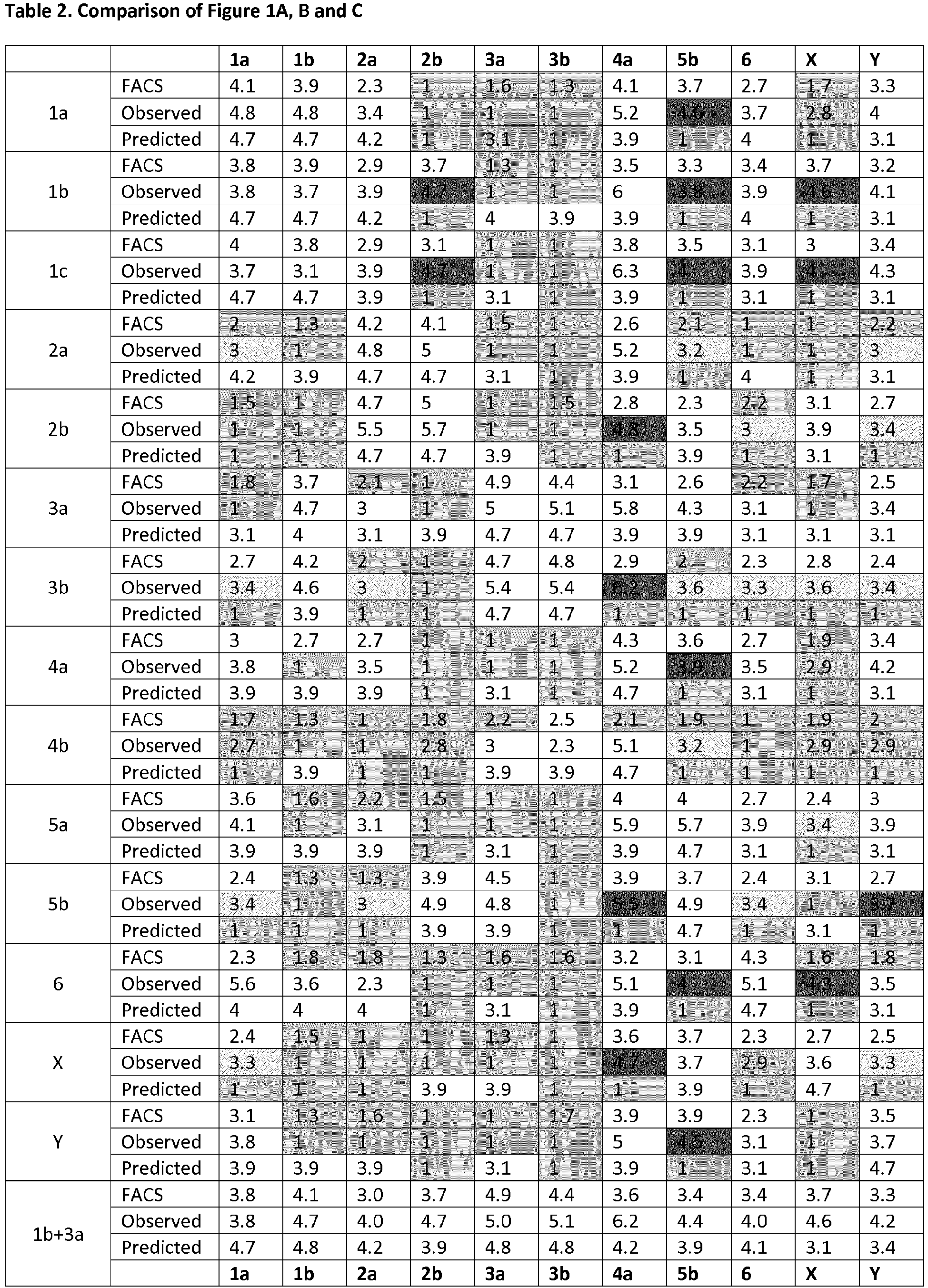

**Table S1- Characterization of S. flexneri strains used in the study by slide agglutination and FACS typing**

| FACS typing and agglutination of *S*. *flexneri* cell lines received from Public Health England with Denka monovalent rabbit typing and grouping sera (only agglutination) to confirm identity of the serotypes. Mean Fluorescence Intensities from 10 to 100 were considered indicative of absence of signal (no binding of indicated antisera to the surface of *S*. *flexneri* serotypes) and correlated with negative agglutination (-) | | | | | | | | | |
|---|---|---|---|---|---|---|---|---|---|
| Serotype | **Type specific serum** Mean Fluorescent Intensity Agglutination strength | | | | | | **Grouping sera** Mean Fluorescent Intensity Agglutination strength | | |
| | **I** | **II** | **III** | **IV** | **V** | **VI** | **3,4** | **6** | **7,8** |
| 1a | 7500 | 30 | 50 | 60 | 60 | 40 - | 7450 | 30 | 40 |
| | +++ | | | - | | | +++ | - | - |
| 1b | 8100 | 50 | 60 | 60 | 50 | 60 | 7200 | 8950 | 30 |
| | +++ | - | - | - | - | - | ++ | ++ | - |
| 1c | 7900 | 60 | 150 | 50 | 60 | 40 | 1550 | 40 | 30 |
| | +++ | - | - | - | - | - | + | - | - |
| 2a | 50 | 9200 | 90 | 60 | 60 | 40 | 5200 | 30 | 25 |
| | - | +++ | - | - | - | - | +++ | - | - |
| 2b | 70 | 8700 | 60 | 60 | 30 | 40 | 30 | 25 | 1530 |
| | - | +++ | | | - | | | - | ++ |
| 3a | 30 | 40 | 8500 | 60 | 30 | 60 | 1200 | 6250 | 3540 |
| | - | - | +++ | - | - | - | +/- | ++ | ++ |
| 3b | 50 | 90 | 7200 | 50 | 40 | 80 | 30 | 4500 | 45 |
| | - | - | +++ | - | - | - | - | ++ | - |
| 4a | 80 | 60 | 30 | 7500 +++ | 30 | 40 | 2350 +++ | 25 | 30 |
| | - | - | - | | - | - | | - | - |
| 4b | 90 | 80 | 60 | 7600 | 30 | 60 | 20 | 1250 | 30 |
| | - | - | - | +++ | - | - | - | ++ | - |
| 5a | 30 | 60 | 40 | 80 | 5800 | 80 | 6500 | 40 | 30 |
| | - | - | - | - | +++ | - | +++ | - | - |
| 5b | 250 | 120 | 170 | 30 | 6600 | 60 | 40 | 30 | 620 |
| | - | - | - | - | +++ | - | - | - | +/- |
| 6 X | 150 | 190 | 120 | 90 | 60 | 2400 | 250 | 30 | 30 |
| | - | - | - | - | - | ++ | +/- | - | - |
| | 80 | 90 | 110 | 60 - | 90 | 60 | 20 | 30 | 450 +/- |
| Y | 150 | 180 | 90 | 90 | 90 | 80 | 540 | 20 | 20 |

**Table S.2- GMMA OAg to Protein ratios**

| **Material** | **ratio w/w OAg/protein** |
|---|---|
| **GMMA-1a** | 0.39 |
| **GMMA-1b** | 0.58 |
| **GMMA-1c** | 0.53 |
| **GMMA-2a** | 0.42 |
| **GMMA-2b** | 0.8 |
| **GMMA-3a** | 0.48 |
| **GMMA-3b** | 0.39 |
| **GMMA-4a** | 0.39 |
| **GMMA-4b** | 0.48 |
| **GMMA-5a** | 0.38 |
| **GMMA-5b** | 0.58 |
| **GMMA-6** | 0.39 |
| **GMMA-X** | 0.12 |
| **GMMA-Y** | 0.46 |

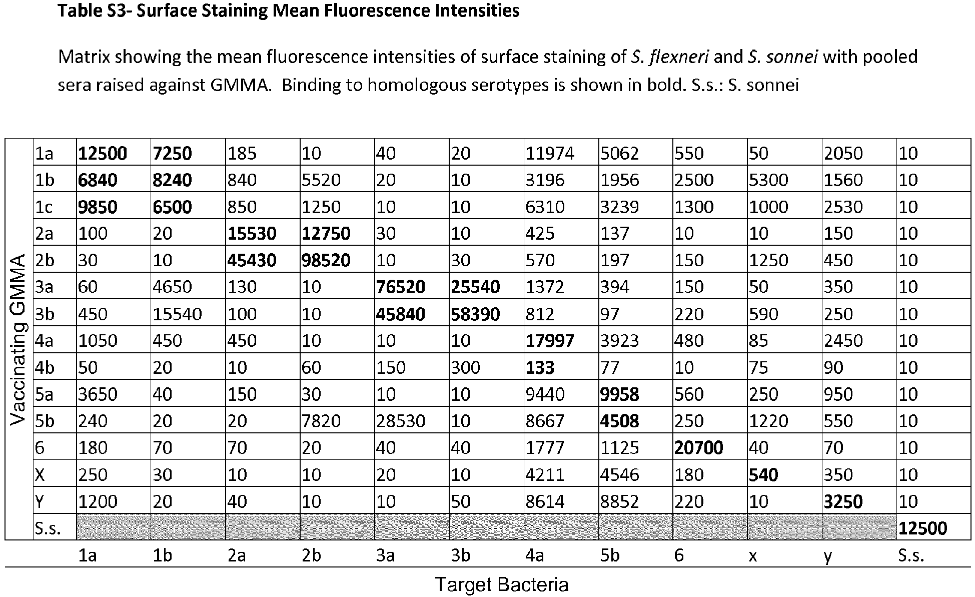

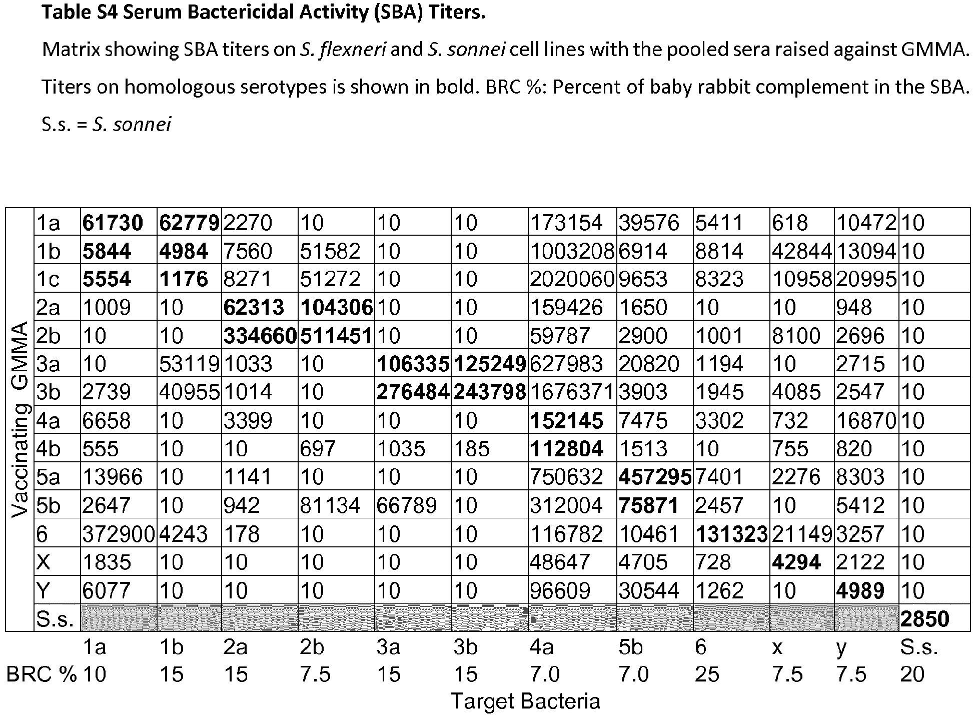

## Claims

1. A *Shigella flexneri* O-antigen of a first serotype or subserotype for use in raising an immune response against one or more *Shigella flexneri* O-antigen of a different serotype or subserotype.

2. The *Shigella flexneri* O-antigen for use according to any preceding claim, wherein the different serotype or subserotype is one or more serotype or subserotype having an SBA score in Table 2 of greater than or equal to 2.3, for example, greater than or equal to 3.0, greater than or equal to 3.6, or greater than or equal to 3.7 and/or wherein the different serotype or subserotype is not one or more serotype or subserotype having an SBA score in Table 2 of less than 3.7, for example, less than 3.6, less than 3.0, or less than 2.3.

3. The *Shigella flexneri* O-antigen for use according to any preceding claim, wherein the first serotype or subserotype is:
1 and the one or more *S. flexneri* O-antigen of a different serotype or subserotype comprises or consists of a serotype or subserotype selected from the group consisting of serotype 1, 2, 3, 4, 5, 6, X and Y, for example, 1, 2, 3, 4, 5, 6, 7 or 8 of the different serotypes;
2 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
3 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
4 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
5 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
6 and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes;
X and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes; and/or
Y and the different serotype or subserotype comprises or consists of a serotype selected from the group consisting of serotype or subserotype 1, 2, 3, 4, 5, 6, X and Y, for example, 1, 2, 3, 4, 5, 6, 7 or 8 of the different serotypes.

4. The *Shigella flexneri* O-antigen for use according to any preceding claim, wherein the first serotype or subserotype is:
1a and the one or more *S. flexneri* O-antigen of a different serotype or subserotype comprises or consists of a serotype or subserotype selected from the group consisting of serotype or subserotype 1b, 2a, 2b, 3a, 3b, 4a, 5b, 6, X and Y, for example, 1, 2, 3, 4, 5, 6, 7, 8, 9 or 10 of the different serotypes or subserotypes;
1b and the one or more *S. flexneri* O-antigen of a different serotype or subserotype comprises or consists of a serotype or subserotype selected from the group consisting of serotype or subserotype 1a, 2a, 2b, 3a, 3b, 4a, 5b, 6, X and Y, for example, 1, 2, 3, 4, 5, 6, 7, 8, 9 or 10 of the different serotypes or subserotypes;
2a and the one or more *S. flexneri* O-antigen of a different serotype or subserotype comprises or consists of a serotype or subserotype selected from the group consisting of serotype or subserotype 1a, 1b, 2b, 3a, 3b, 4a, 5b, 6, X and Y, for example, 1, 2, 3, 4, 5, 6, 7, 8, 9 or 10 of the different serotypes or subserotypes;
2b and the one or more *S. flexneri* O-antigen of a different serotype or subserotype comprises or consists of a serotype or subserotype selected from the group consisting of serotype or subserotype 1a, 1b, 2a, 3a, 3b, 4a, 5b, 6, X and Y, for example, 1, 2, 3, 4, 5, 6, 7, 8, 9 or 10 of the different serotypes or subserotypes;
3a and the one or more *S. flexneri* O-antigen of a different serotype or subserotype comprises or consists of a serotype or subserotype selected from the group consisting of serotype or subserotype 1a, 1b, 2a, 2b, 3b, 4a, 5b, 6, X and Y, for example, 1, 2, 3, 4, 5, 6, 7, 8, 9 or 10 of the different serotypes or subserotypes;
3b and the one or more *S. flexneri* O-antigen of a different serotype or subserotype comprises or consists of a serotype or subserotype selected from the group consisting of serotype or subserotype 1a, 1b, 2a, 2b, 3a, 4a, 5b, 6, X and Y, for example, 1, 2, 3, 4, 5, 6, 7, 8, 9 or 10 of the different serotypes or subserotypes;
4a and the one or more *S. flexneri* O-antigen of a different serotype or subserotype comprises or consists of a serotype or subserotype selected from the group consisting of serotype or subserotype 1a, 1b, 2a, 2b, 3a, 3b, 5b, 6, X and Y, for example, 1, 2, 3, 4, 5, 6, 7, 8, 9 or 10 of the different serotypes or subserotypes;
5b and the one or more *S. flexneri* O-antigen of a different serotype or subserotype comprises or consists of a serotype or subserotype selected from the group consisting of serotype or subserotype 1a, 1b, 2a, 2b, 3a, 3b, 4a, 6, X and Y, for example, 1, 2, 3, 4, 5, 6, 7, 8, 9 or 10 of the different serotypes or subserotypes;
6 and the one or more *S. flexneri* O-antigen of a different serotype or subserotype comprises or consists of a serotype or subserotype selected from the group consisting of serotype or subserotype 1a, 1b, 2a, 2b, 3a, 3b, 4a, 5b, X and Y, for example, 1, 2, 3, 4, 5, 6, 7, 8, 9 or 10 of the different serotypes or subserotypes;
X and the one or more *S. flexneri* O-antigen of a different serotype or subserotype comprises or consists of a serotype or subserotype selected from the group consisting of serotype or subserotype 1a, 1b, 2a, 2b, 3a, 3b, 4a, 5b, 6, and Y, for example, 1, 2, 3, 4, 5, 6, 7, 8, 9 or 10 of the different serotypes or subserotypes; and/or
Y and the one or more *S. flexneri* O-antigen of a different serotype or subserotype comprises or consists of a serotype or subserotype selected from the group consisting of serotype or subserotype 1a, 1b, 2a, 2b, 3a, 3b, 4a, 5b, 6 and X, for example, 1, 2, 3, 4, 5, 6, 7, 8, 9 or 10 of the different serotypes or subserotypes.

5. The *Shigella flexneri* O-antigen for use according to any preceding claim, wherein the first serotype or subserotype is from another serotype to the different serotype or subserotype, for example:
where the first serotype or subserotype is 1, the different serotype or subserotype is not, or is not a subserotype of, serotype 1;
where the first serotype or subserotype is 2, the different serotype or subserotype is not, or is not a subserotype of, serotype 2;
where the first serotype or subserotype is 3, the different serotype or subserotype is not, or is not a subserotype of, serotype 3;
where the first serotype or subserotype is 4, the different serotype or subserotype is not, or is not a subserotype of, serotype 4;
where the first serotype or subserotype is 5, the different serotype or subserotype is not, or is not a subserotype of, serotype 5;
where the first serotype or subserotype is 6, the different serotype or subserotype is not, or is not a subserotype of, serotype 6;
where the first serotype or subserotype is X, the different serotype or subserotype is not, or is not a subserotype of, serotype X;
where the first serotype or subserotype is Y, the different serotype or subserotype is not, or is not a subserotype of, serotype Y;

6. The *Shigella flexneri* O-antigen for use according to any preceding claim, wherein the first serotype or subserotype is:
2a, the different serotype or subserotype is not subserotype 1b;
2a, the different serotype or subserotype is not subserotype 2b;
2a, the different serotype or subserotype is not subserotype 5b;
2a, the different serotype or subserotype is not subserotype Y;
3a, the different serotype or subserotype is not subserotype 1b;
3a, the different serotype or subserotype is not subserotype 2b;
3a, the different serotype or subserotype is not subserotype 5b;
3a, the different serotype or subserotype is not, or is not a subserotype of, serotype Y;
2a, and is provided in combination with an additional O-antigen of serotype or subserotype 3a, the different serotype or subserotype is not subserotype 1b;
2a, and is provided in combination with an additional O-antigen of serotype or subserotype 3a, the different serotype or subserotype is not subserotype 2b;
2a, and is provided in combination with an additional O-antigen of serotype or subserotype 3a, the different serotype or subserotype is not subserotype b5;
2a, and is provided in combination with an additional O-antigen of serotype or subserotype 3a, the different serotype or subserotype is not, or is not a subserotype of, serotype Y; and/or
2a, the different serotype or subserotype is not, or is not a subserotype of, serotype 6;
Y, the different serotype or subserotype is not, or is not subserotype 1b; and or
Y, the different serotype or subserotype is not, or is not subserotype 2a.

7. The *Shigella flexneri* O-antigen for use according to any preceding claim, wherein the first serotype or subserotype is:
serotype 1 and the different serotype or subserotype is one or more serotype selected from the group consisting of 2, 5 and X, for example, 1, 2 or 3 of the different serotypes.
serotype 2 and the different serotype or subserotype is one or more serotype selected from the group consisting of 4, 5, 6 and Y, for example, 1, 2 or 3 of the different serotypes.
serotype 3 and the different serotype or subserotype is one or more serotype selected from the group consisting of 1, 2, 4, 5, 6, X and Y, for example, 1, 2, 3, 4, 5, 6 or 7 of the serotypes;
serotype 4 and the different serotype or subserotype is one or more serotype selected from the group consisting of 1, 2, 5, X and Y, for example, 1, 2, 3, 4 or 5 of the serotypes;
serotype 5 and the different serotype or subserotype is one or more serotype selected from the group consisting of 1, 2, 4, 6, X and Y, for example, 1, 2, 3, 4, 5 or 6 of the serotypes;
serotype 6 and the different serotype or subserotype is one or more serotype selected from the group consisting of 5 and X, for example, 1 or 2 of the serotypes;
serotype X and the different serotype or subserotype is one or more serotype selected from the group consisting of 1, 4, 6 and Y, for example, 1, 2, 3 or 4 of the serotypes; and/or
serotype Y and the different serotype or subserotype is 5.

8. The *Shigella flexneri* O-antigen for use according to any preceding claim, wherein the first serotype or subserotype is:
subserotype 1a and the different serotype or subserotype is one or more subserotype selected from the group consisting of 5b and X, for example, 1 or 2 of the subserotypes;
subserotype 1b and the different serotype or subserotype is one or more subserotype selected from the group consisting of 2b, 5b and X, for example, 1, 2 or 3 of the subserotypes;
subserotype 1c and the different serotype or subserotype is one or more subserotype selected from the group consisting of 2b, 5b and X, for example, 1, 2 or 3 of the serotypes;
subserotype 2a and the different serotype or subserotype is subserotype 1a, 5b and Y, for example, 1, 2 or 3 of the subserotypes;
subserotype 2b and the different serotype or subserotype is one or more subserotype selected from the group consisting of 4a, 6 and Y, for example, 1, 2 or 3 of the subserotypes;
subserotype 3b and the different serotype or subserotype is one or more subserotype selected from the group consisting of 1a, 2a, 4a, 5b, 6, X and Y, for example, 1, 2, 3, 4, 5, 6 or 7 of the subserotypes;
subserotype 4a and the different serotype or subserotype is one or more subserotype selected from the group consisting of 5b and X, for example, 1 or 2 of the subserotypes;
subserotype 4b and the different serotype or subserotype is one or more subserotype selected from the group consisting of 1a, 2b, 5b, X and Y for example, 1, 2, 3, 4 or 5 of the subserotypes.
subserotype 5a and the different serotype or subserotype is X;
the first serotype is subserotype 5b and the different serotype or subserotype is one or more subserotype selected from the group consisting of 1a, 2a, 4a, 6 and Y, for example, 1, 2, 3, 4 or 5 of the subserotypes;
serotype 6 and the different serotype or subserotype is one or more subserotype selected from the group consisting of 5b and X, for example, 1 or 2 of the subserotypes;
serotype X and the different serotype or subserotype is one or more serotype selected from the group consisting of 1a, 4a, 6 and Y, for example, 1, 2, 3 or 4 of the subserotypes; and/or
serotype Y and the different serotype or subserotype is subserotype 5b.

9. The *Shigella flexneri* O-antigen for use according to claim any preceding claim, wherein the first serotype or subserotype is:
subserotype 1b and the different serotype or subserotype is not one or more subserotype selected from the group consisting of 3a and 3b, for example, 1 or 2 of the subserotypes;
subserotype 1c and the different serotype or subserotype is not 3a;
subserotype 2a and the different serotype or subserotype is not one or more subserotype selected from the group consisting of 3a and 6, for example, 1 or 2 of the subserotypes;
subserotype 2b and the different serotype or subserotype is not 3a;
subserotype 3a and the different serotype or subserotype is not one or more subserotype selected from the group consisting of 1a, 2b and X, for example, 1, 2 or 3 of the subserotypes;
subserotype 4a and the different serotype or subserotype is not one or more subserotype selected from the group consisting of 1b and 3a, for example, 1 or 2 of the subserotypes;
subserotype 4b and the different serotype or subserotype is not 1b.
subserotype 5a and the different serotype or subserotype is not one or more subserotype selected from the group consisting of 1b and 3a, for example, 1 or 2 of the subserotypes;
the first serotype is subserotype 5b and the different serotype or subserotype is not X;
serotype 6 and the different serotype or subserotype is not X;
serotype X and the different serotype or subserotype is not one or more serotype selected from the group consisting of 2b and 3a, for example, 1 or 2 of the subserotypes; and/or
serotype Y and the different serotype or subserotype is not one or more serotype selected from the group consisting of 1b, 2a and 3a, for example, 1, 2 or 3 of the subserotypes.

10. The *Shigella flexneri* O-antigen for use according to any preceding claim, wherein the O-antigen of a first serotype is provided in combination with one or more O-antigen of a further serotype or subserotype, for example 1, 2, 3, 4, 5, 6, 7, 8, 9, 10, 11, 12, 13, 14 or 15 O-antigen further serotypes or subserotypes, for example:
wherein first and further subserotypes comprise or consist of combinations selected from the group consisting of:
a. 1b and 3a;
b. 1b and 3b;
c. 1c and 3a;
d. 1c and 3b;
e. 2a and 3b;
f. 3a and 4b; and
g. 3b and 5b.

11. The *Shigella flexneri* O-antigen for use according to any preceding claim, wherein one or more of the different serotype(s) or subserotype(s) is not provided, for example, one or more of 1a, 1b, 1c (or 7a), 1d, 2a, 2b, 3a, 3b, 4a, 4av, 4b, 5a, 5b, X, Xv, Y, Yv, 6 and 7b is not provided, for example, 1, 2, 3, 4, 5, 6, 7, 8, 9, 10, 11, 12, 13, 14, 15, 16, 17 or 18 of the different serotype(s) or subserotype(s) is not provided, and (optionally), wherein the *Shigella flexneri* O-antigen for use is capable of raising an immune response against one or more of the different serotype(s) or subserotype(s) that is not provided, e.g.:
wherein serotype 1 (for example, 1a, 1b, or 1c) is provided and serotype 6 is not provided and/or
wherein serotype 3 (for example, 3a, 3b, or 3c) is provided and serotype 6 is not provided.

12. The *Shigella flexneri* O-antigen for use according to any preceding claim, wherein O-antigen from one or more *Shigella* species other than *Shigella flexneri* is provided in combination with the O-antigen of a first serotype, for example, wherein the one or more other *Shigella* species is selected from the group consisting of:
a. *Shigella sonnei;*
b. *Shigella boydii* (for example, serotype 1, 2, 3, 4, 5, 6, 7, 8, 9, 10, 11, 12, 13, 14, 15, 16, 17, 18, 19 or 20); and
c. *Shigella dysenteriae* (for example, serotype 1, 2, 3, 4, 5, 6, 7, 8, 9, 10, 11, 12, 13, 14 or 15).

13. The *Shigella flexneri* O-antigen for use according to any preceding claim, wherein the first serotype or subserotype and/or other *Shigella* species is/are provided:
a. unassociated with another macromolecule;
b. as a component of lipopolysaccharide (LPS), or a fragment thereof; or
c. conjugated to another macromolecule, for example, a protein (e.g., a carrier protein such as CRM197, tetanus toxoid, meningococcal outer membrane protein complex (OMPC), diphtheria toxoid, and *H. influenzae* protein D).

14. The *Shigella flexneri* O-antigen for use according to any one of claims 45-47, wherein the membrane component is a component of an OMV selected from the group consisting of a detergent-extracted OMV (dOMV); or native OMV (nOMV).

15. The *Shigella flexneri* O-antigen for use according to any preceding claim, wherein the immune response comprises or consists of a protective immune response, e.g., an *in vitro* protective immune response and/or an *in vivo* protective immune response.

16. A binding moiety capable of specifically binding to one or more O-antigen defined in any one of claims 1-15.

17. A pharmaceutical composition comprising: an O-antigen for use defined in claims 1-15 and/or a binding moiety as defined in claim 16.

18. A kit comprising or consisting of an O-antigen for use defined in claims 1-15, a binding moiety as defined in claim 16 and/or a pharmaceutical composition as defined in claim 17; and (optionally) instructions for use.

19. An O-antigen for use defined in claims 1-15, a binding moiety as defined in claim 16, a pharmaceutical composition as defined in claim 17, and/or a kit as defined in claim 18, for use in medicine.

20. The use of a binding moiety as defined in claim 16, for detecting the presence of bacteria, for example, wherein the bacteria are one or more bacterium defined in any one of the preceding claims.
